# EUROPÄISCHE PATENTSCHRIFT

(11) **EP 3 008 036 B1**
(45) Veröffentlichungstag und Bekanntmachung des Hinweises auf die Patenterteilung: **02.08.2017**
(21) Anmeldenummer: 14733160.7
(22) Anmeldetag: 25.06.2014
(51) Int. Cl.: B01D 11/04, B01J 19/18, C07C 201/02, C07C 203/04

(54) **VERFAHREN UND VORRICHTUNG ZUR ENTFERNUNG VON BEI DER HERSTELLUNG VON ALIPHATISCHEN NITRATESTERN ANFALLENDEN VERUNREINIGUNGEN**
METHOD AND DEVICE FOR REMOVING CONTAMINANTS ARISING DURING THE PRODUCTION OF ALIPHATIC NITRATE ESTERS
PROCÉDÉ ET DISPOSITIF PERMETTANT D'ÉLIMINER LES IMPURETÉS RÉSULTANT DE LA PRODUCTION D'ESTERS DE NITRATE ALIPHATIQUES

(30) Priorität: 15.08.2013 DE 102013013474; 02.10.2013 DE 102013110952
(43) Veröffentlichungstag der Anmeldung: 20.04.2016
(73) Patentinhaber: Josef Meissner GmbH & Co. KG, 50968 Köln (DE)
(72) Erfinder: PÖHLMANN, Jürgen, 50969 Köln (DE); HERMANN, Heinrich, 50858 Köln (DE); HÄNDEL, Mirko, 53819 Neunkirchen-Seelscheid (DE); GEBAUER, Jürgen, 53840 Troisdorf (DE)
(74) Vertreter: Strehlke, Ingo Kurt
(86) Internationale Anmeldenummer: PCT/EP2014/063334
(87) Internationale Veröffentlichungsnummer: WO 2015/022110

(56) Entgegenhaltungen:
- WO-A1-2012/156095
- DE-B- 1 039 049
- US-A- 837 044

## Beschreibung

Die vorliegende Erfindung betrifft das technische Gebiet der Herstellung von Salpetersäureestern (synonym auch als Nitroester oder Nitratester bezeichnet) ein- oder mehrwertiger (cyclo)aliphatischer Alkohole (d. h. also mit anderen Worten die Nitrierung ein- oder mehrwertiger aliphatischer bzw. cycloaliphatischer Alkohole zu den entsprechenden Salpetersäureestern), insbesondere die Aufreinigung der nach Abtrennung der Nitrierendsäure anfallenden nitrierten Rohsalpetersäureester.

Insbesondere betrifft die vorliegende Erfindung ein Verfahren zur Entfernung von bei der Herstellung von aliphatischen oder cycloaliphatischen Salpetersäureestern anfallenden Verunreinigungen (wie z. B. insbesondere nicht umgesetzte Edukte, Reaktionsnebenprodukte, Nitriersäure und deren Umsetzungsprodukte, wie Stickoxide oder Salpetrige Säure, etc.), insbesondere ein Verfahren zur Entfernung von Verunreinigungen aus bei der Nitrierung von nitrierbaren ein- oder mehrwertigen aliphatischen oder cycloaliphatischen Alkoholen nach Abtrennung der Nitrierendsäure anfallenden nitrierten Rohsalpetersäureestern.

Des Weiteren betrifft die vorliegende Erfindung die Verwendung einer Vorrichtung bzw. Anlage zur Entfernung von bei der Herstellung von aliphatischen oder cycloaliphatischen Salpetersäureestern anfallenden Verunreinigungen, insbesondere die Verwendung einer Vorrichtung bzw. Anlage zur Entfernung von Verunreinigungen aus bei der Nitrierung von nitrierbaren ein- oder mehrwertigen aliphatischen oder cycloaliphatischen Alkoholen nach Abtrennung der Nitrierendsäure anfallenden nitrierten Rohsalpetersäureestern.

Schließlich betrifft die vorliegende Erfindung die Verwendung einer Produktionsanlage zur Nitrierung nitrierbarer aliphatischer oder cycloaliphatischer Alkohole mit nachfolgender Aufreinigung der nitrierten Rohprodukte (d. h. mit nachfolgender Aufreinigung der resultierenden Rohsalpetersäureester) bzw. die Verwendung einer Produktionsanlage zur Herstellung von Salpetersäureestern ein- oder mehrwertiger aliphatischer oder cycloaliphatischer Alkohole mit nachfolgender Aufreinigung der nitrierten Rohprodukte.

Aliphatische Nitroester (synonym auch als aliphatische Salpetersäureester, aliphatische Nitratester oder dergleichen bezeichnet), wie z. B. Pentaerythrittetranitrat (PETN), Glycerintrinitrat (NGL), Ethylenglykoldinitrat (EGDN), Ethylhexylnitrat (EHN) etc., werden im Allgemeinen in einer kontinuierlich betriebenen Anlage (z. B. im Fall von NGL, EGDN oder deren Mischungen nach den Verfahren nach Schmid-Meissner oder Biazzi, nach dem Injektorverfahren oder nach dem N.A.B.-Prozess von Nielsen und Brunnberg oder im Fall von EHN nach einem modifizierten Verfahren gemäß EP 1 792 891 A1) durch Umsetzung des entsprechenden Alkohols (wie z. B. Pentaerythrit, Glycerin, Ethylenglykol, Ethylhexylethanol etc.) mit Salpetersäure direkt bzw. allein oder aber in Gegenwart von Schwefelsäure als Katalysator und wasserbindendem Mittel hergestellt. Die Nitrierung der Alkohole (d. h. deren Umsetzung zu Salpetersäureestern) erfolgt im Allgemeinen in der flüssigen Phase, und zwar entweder als Reinsubstanz oder aber als Gemisch oder aber in Lösung in inerten, nicht mit Wasser mischbaren Lösungsmitteln.

Die resultierenden rohen Nitroester (nachfolgend synonym auch als Rohsalpetersäureester, Rohnitratester, nitrierte Rohprodukte oder dergleichen bezeichnet) müssen vor ihrer Weiterverarbeitung einer mehrstufigen Wäsche und zusätzlichen Reinigungsstufen unterworfen werden, um die in den rohen Nitroestern noch gelösten oder suspendierten Verunreinigungen, wie z. B. Schwefelsäure, Salpetersäure, Nitrose etc., und Nebenprodukte aus dem oxidativen Abbau der Ausgangsalkohole, zu entfernen und um damit die Stabilität der Nitroester, welche zum großen Teil hochempfindliche Explosivstoffe darstellen, zu verbessern.

Üblicherweise besteht die Wäsche der rohen Nitroester zur Entfernung der darin gelösten und suspendierten Säuren des Nitriergemisches und anderer saurer oder anderweitiger mit dem Waschmittel extrahierbarer Verunreinigungen aus drei Schritten (siehe z. B. T. Urbanski, Vol. 2, Seiten 97 ff., insbesondere Figs. 43, 44, 59 und 60, Pergamon Press, Reprint 1985). Als Waschmedium wird dafür üblicherweise Wasser eingesetzt. Die Wäsche wird im Allgemeinen als flüssig/flüssig-Wäsche mit dem bei Waschtemperatur flüssigen Nitroester oder als Lösung desselben in einem geeigneten Lösungsmittel durchgeführt.

Diese dreistufige Wäsche der nitrierten Rohprodukte (d. h. der rohen Nitro- bzw. Salpetersäureester) umfasst üblicherweise die folgenden Schritte:
1. eine saure Wäsche ("saure Wäsche") mit Wasser zur Entfernung der gelösten und suspendierten Mineralsäuren, wie z. B. Schwefelsäure, Salpetersäure und Nitrose;
2. eine basische bzw. alkalische Wäsche ("basische Wäsche" oder "Alkaliwäsche") in Gegenwart einer Base, wie z. B. Natriumcarbonat (Soda), Natriumbicarbonat, Ammoniak, Natronlauge, Kalilauge etc., insbesondere zur Entfernung der im Nitratester nach der ersten Wäsche noch vorhandenen Mineralsäuren und Reste von Nitrose und anderen schwach acide Verunreinigungen aus einer oxidativen Zersetzung des eingesetzten Alkohols oder anderen aliphatischen oder cyclischen Kohlenwasserstoffen, die als Spuren im Ausgangsalkohol vorhanden waren;
3. eine Wäsche mit Wasser ("Neutralwäsche") zur Entfernung der Restspuren von Alkali und der weiteren Reduzierung der noch in Spuren im Produkt verbliebenen Verunreinigungen.

Aber auch eine Wäsche der rohen Nitratester direkt mit Sodalösung, d. h. unter Auslassung der Waschstufe "saure Wäsche", wird teilweise praktiziert und ist Stand der Technik (siehe z. B. T. Urbanski, Vol. 2, Biazzi-Process, Seiten 107 ff., insbesondere Fig. 48, Pergamon Press, Reprint 1985; T. Urbanski, Vol. 4, Seite 328, Pergamon Press 1984; B. Brunnberg, Industrial and Laboratory Nitration, ACS Symposium Series No. 22 (Editors: L .F. Albright and H. J. T. Hanson), Seite 341, Washington D.C., 1976).

Ziel dieser Waschschritte ist es, neben einem reinen Produkt mit hoher Stabilität möglichst wenig Abwasser pro Tonne Produkt zu erhalten, wobei im Abwasser die ausgewaschenen Verunreinigungen und die entsprechend ihrer Löslichkeit noch vorhandenen Nitratester(spuren) so vorliegen sollen, dass ihre Entsorgung kostengünstig durchgeführt werden kann.

Zur Minimierung der für diese Wäsche benötigten Wassermengen und zur Rückgewinnung der in den rohen Nitratestern in beträchtlichen Mengen gelösten Säuren, vor allem Salpetersäure, kann - wie z. B. in DE-PS 505 424 und DE-PS 546 718 beschrieben - die Wäsche mit einer reduzierten Waschwassermenge im ersten Waschschritt ("saure Wäsche") derart durchgeführt werden, dass eine Waschsäure mit einem Gehalt an Salpetersäure von 20- bis 60%-iger Stärke bzw. von 35 bis 55 % und bis zu 15 % Schwefelsäure erhalten wird. Diese Waschsäure kann direkt oder zusammen mit der Nitrierendsäure aufgearbeitet werden.

Weiterhin kann die Wäsche beispielsweise im Gegenstrom derart erfolgen, dass das Waschwasser aus dem dritten Waschschritt ("Neutralwäsche") direkt oder nach Zusatz von Basen zur Wäsche des Nitratesters in den zweiten Waschschritt ("Alkaliwäsche") eingesetzt wird und dieses Waschwasser aus dem zweiten Waschschritt (d. h. der alkalischen Waschstufe) in den ersten Waschschritt (d. h. die saure Wäsche) eingeführt wird (vgl. DE-PS 505 424 und DE-AS 1 135 876).

Üblich ist es, dass bei diesen drei Waschschritten aus Gründen der Sicherheit bevorzugt Injektoren (Strahlpumpen) als Förderer mit dem Waschmedium als Treibmittel zwischen den einzelnen Waschschritten eingesetzt werden und die eigentliche Wäsche in mit Luft betriebenen Waschkolonnen erfolgt. Nach beendeter Wäsche wird der gereinigte Nitratester als Emulsion mit Wasser in ein Lager oder zur Weiterverarbeitung transportiert.

In der DE-PS 710 826 wird ein Verfahren beschrieben, bei dem die dreistufige Wasche von Nitratestern mehrwertiger Alkohole im Gegenstrom in mit Luft gerührten Waschkolonnen durchgeführt wird, wobei die Nitratester mit der Waschflüssigkeit in Injektoren vorgemischt werden und diese vorgemischte Emulsion über Luftinjektoren in die Waschkolonne eingespeist wird. Am Ende der Wäsche wird der gewaschenen Nitratester nach Phasentrennung mittels Injektoren, welche mit im Kreis geführtem Treibwasser betrieben werden, zum Lager transportiert. Auch bei den in T. Urbanski, Vol. 2, Seiten 97 ff., insbesondere Figs. 59 und 60, Pergamon Press, Reprint 1985, dokumentierten Waschverfahren kommt das in der DE-PS 710 826 beschriebene Waschverfahren in Verbindung mit dem Injektorverfahren zum Einsatz.

Für eine Entfernung eines Stoffes aus einem Stoffgemisch durch Extraktion/ Wäsche in einem mit diesem Stoffgemisch nicht mischbaren Stoff, wie im vorliegenden Fall des Gemisches flüssiger NitratesterNerunreinigungen mit Wasser, ist es für eine erfolgreiche Extraktion der Verunreinigungen aus dem Stoffgemisch erforderlich, die beiden nicht miteinander mischbaren Phasen so ineinander zu verteilen - sei es als Öl-in-Wasser-Emulsion (O/W-Emulsion), sei es als Wasser-in-Öl-Emulsion (W/O-Emulsion) -, dass über eine genügend lange Zeit eine derart große Austauschfläche zwischen den nicht miteinander mischbaren Phasen vorliegt, dass das Verteilungsgleichgewicht für die zu extrahierenden Stoffe erreicht wird und die extrahierten Stoffe in der Extraktphase noch durch nachfolgende Umsetzungen so verändert werden können, dass eine Rückextraktion nicht mehr möglich ist.

Es ist bekannt, dass Injektoren oder Strahlsauger geeignet sind, miteinander mischbare Flüssigkeiten zu mischen, dass aber Injektoren als Dispergierorgan für zwei miteinander nicht mischbare Flüssigkeiten ineinander nicht geeignet sind. Für eine gute Wäsche sind die im Injektor erzeugten Tropfengrößen nicht klein genug. Die Stabilität der erzeugten Emulsion ist zu gering, und die für den optimalen Stoffaustausch benötigte Austauschfläche ist - in Verbindung mit der kurzen Verweilzeit im Mischrohr des Injektor (ca. 1 Sekunde) - damit zu klein.

Die mit Injektoren erzeugten, wenig stabilen Nitratesteremulsionen in Wasser neigen daher zu einer schnellen Phasentrennung. Dies ist besonders problematisch für den Transport solcher Emulsionen über längere Strecken. Wie in der DE-PS 973 718 anschaulich beschrieben, findet - sofern eine Emulsion für sich allein gefördert wird - in den langen, wenig geneigten Förderrohren leicht eine Auftrennung in Sprengöl und Wasser statt. Es kann eine zusammenhängende Sprengölschicht entstehen, welche ein Durchdetonieren ermöglicht.

Um die Koaleszenz der Nitratester nach dem Verlassen des Injektors zu unterbinden und speziell beim Emulsionstransport eine Durchdetonation durch abgeschiedenes Sprengöl zu verhindern, werden - wie beispielsweise in der US 2 140 897 A, der DE-PS 820 575, der DE-PS 973 718, der DE-AS 1 058 093, der DE-OS 1 571 221 oder der DE-OS 2 055 093 beschrieben - Verfahren angegeben, welche es gestatten, die Emulsion des Nitratesters in Wasser zu stabilisieren bzw. die Bildung von zur Durchdetonation fähigen Stromfäden aus abgeschiedenen Nitratestern zu unterbinden. Dies geschieht bevorzugt durch Zumischen von Luft oder durch Reemulgieren mit Luft oder aber durch Unterbrechen des Emulsionsstromes mit Wasser oder Luft.

Es ist aber bekannt, dass Gasblasen im flüssigen Nitratester nicht nur dessen Schlagempfindlichkeit, verursacht durch adiabatische Kompression, wesentlich erhöhen können (vgl. DE-OS 1 571 221), sondern dass die Zweiphasengemische aus zusätzlich mitangesaugter Luft, wie bereits in der DE-AS 1 058 039 beschrieben, zu derart stabilen Emulsionen führen können, dass nur durch Einsatz von Zentrifugalseparatoren eine Phasentrennung möglich ist.

Diese Gefahr eines Lufteinschlusses in den flüssigen Nitratester kann durch die in der DE-OS 1 571 221 und der DE-OS 2 055 093 beschriebenen Maßnahmen unterbunden werden. Durch die Unterbrechung der homogenen Emulsionssäule in der Transportleitung durch eine nitratesterfreie Wassersäule wird die Ausbildung eines durchdetonationsfähigen Sprengstofffadens durch zu schnelle Koaleszenz des im Injektor emulgierten Nitratesters wirkungsvoll unterbunden.

Die durch den Injektor mit seiner relativ geringen Dispergierleistung erzeugten wenig stabilen Dispersionen von Nitratester in Waschwasser oder umgekehrt und die infolgedessen nicht optimale Austauschfläche für eine effektive Wäsche (zusätzlich in Verbindung mit den sehr kurzen Verweilzeiten im Mischbereich des Injektors) erlauben eine nur unvollständige Entfernung der Verunreinigungen im zu reinigenden Nitratester. Um die gewünschten Reinigungsgrade zu erreichen, werden die Injektoren deshalb mit Verweilzeitbehältern mit aktiver Dispergierung gekoppelt.

Als Waschapparate werden daher für die Wäsche der zu reinigenden Nitroester auf den einzelnen Waschstufen üblicherweise mit Luft betriebene Waschkolonnen (DE-PS 710 826) in Verbindung mit Injektoren als Transportmittel oder Rührkesselkaskaden in Verbindung mit Injektoren (T. Urbanski, Vol. 4, Seite 328, insbesondere Fig. 46, Pergamon Press 1984) eingesetzt. Der Einsatz der mit Luft gerührten Waschkolonnen ist aufwendig und teuer und entspricht nicht mehr heutigen Sicherheitsvorstellungen, nach denen die Gegenwart von Luftblasen im Nitratester vermieden werden soll (DE-OS 1 571 221). Auch mehrstufige Rührkesselkaskaden sind aufwendig und teuer.

Auch der Einsatz von Injektoren allein, d. h. ohne Verweilzeitbehälter, wie in DE-AS 1 039 049 vorgeschlagen, ist aufwendig und teuer. Um mit Injektoren ohne Verweilzeitbehälter und zusätzlichen Dispergiereinrichtungen eine optimale Reinigung zu erreichen, muss mehrstufig in jedem Waschschritt gearbeitet werden, damit die für die Weiterverarbeitung des Nitratester gewünschte Reinheit und Stabilität erhalten werden kann.

Der Emulsionstransport von Nitratestern, wie in der DE-OS 1 571 221 und in der DE-OS 2 055 093 beschrieben, ist als zusätzliche Waschstufe wenig geeignet, weil er wegen seiner durch einen Injektor erzeugten wenig stabilen Emulsion mit relativ großen Tropfen nur höchstens eine polierende Wirkung besitzt, um Spuren an Waschmedium zu entfernen, welche als Mikroemulsion aus den vorgelagerten Waschstufen noch mitgeschleppt wurden.

Dokument DE 10 39 049 B betrifft ein kontinuierliches Verfahren zur Herstellung von Salpetersäureestern und organischen Nitroverbindungen durch Nitrierung mit Nitriersäure, wobei die Reaktionsbestandteile mit einem für die fragliche Nitrierreaktion geeigneten Wärmegehalt zugeführt und in einem Injektor unter Vermischen zur Reaktion gebracht werden, worauf die Reaktionsmischung in einem Abscheider übergeführt wird, in welchem die erzeugten Nitroprodukte von der überschüssigen Säure getrennt werden, wobei die Vermischung in einem Injektor erfolgt, in welchen die Nitriersäure unter Druck eingeführt wird, wobei der andere Reaktionsbestandteil aus einem Vorratsbehälter in den Injektor gesaugt wird, und zwar unter solchen Bedingungen, dass die Mischung der Reaktionsbestandteile und die Reaktion momentan und im Wesentlichen vollständig unmittelbar im Injektor und somit in einem sehr begrenzten Raum stattfindet.

Dokument WO 2012/156095 A1 betrifft ein Verfahren zur Entfernung von Verunreinigungen aus bei der Nitrierung von nitrierbaren aromatischen Verbindungen nach Abtrennung der Nitrierendsäure anfallenden nitrierten Rohprodukte durch Behandlung mit einem Waschmedium sowie eine zur Durchführung dieses Verfahrens geeignete Anlage bzw. Vorrichtung und eine entsprechende Produktionsanlage zur Nitrierung nitrierbarer aromatischer Verbindungen mit nachfolgender Aufreinigung der nitrierten Produkte.

Insgesamt arbeiten die aus dem Stand der Technik bekannten Verfahren und Anlagen zur Reinigung von Nitratestern entweder nicht mit hoher Effizienz, oder aber sie sind unter dem Gesichtspunkt moderner sicherheitstechnischer Standards nicht mehr zufriedenstellend.

Der vorliegenden Erfindung liegt daher die Aufgabe zugrunde, ein Verfahren und eine Vorrichtung zur Entfernung von bei der Herstellung von aliphatischen Nitratestern anfallenden Verunreinigungen, insbesondere zur Entfernung von Verunreinigungen aus bei der Nitrierung von nitrierbaren ein- oder mehrwertigen aliphatischen Alkoholen nach Abtrennung der Nitrierendsäure anfallenden nitrierten Rohnitratestern, sowie eine (Produktions-)Anlage zur Nitrierung nitrierbarer ein- oder mehrwertiger aliphatischer Alkohole mit nachfolgender Aufreinigung der nitrierten Salpetersäureester bereitzustellen, wobei die zuvor geschilderten, im Zusammenhang mit dem Stand der Technik auftretenden Probleme und Nachteile zumindest weitgehend vermieden werden sollen.

Insbesondere ist eine Aufgabe der vorliegenden Erfindung darin zu sehen, ein Verfahren und eine zur Durchführung dieses Verfahrens geeignete Vorrichtung bzw. Anlage bereitzustellen, mit denen eine effiziente Aufreinigung der Nitratester, wie sie aus der Nitrierung von ein- oder mehrwertigen Alkoholen nach Abtrennung der sogenannten Nitrierendsäuren hervorgehen, ermöglicht werden kann.

Weiterhin besteht eine Aufgabe der vorliegenden Erfindung darin, die Wäsche der nach Abtrennung der Nitrierendsäure resultierenden rohen Nitratester oder deren Lösungen in inerten Lösungsmitteln, in denen signifikante Mengen an Verunreinigungen, wie beispielsweise noch mitgerissene Nitriersäure, gelöste Schwefelsäure, Salpetersäure, Nitrose, Abbauprodukte aus dem oxidativen Abbau der Ausgangsalkohole etc. vorliegen können, sozusagen einstufig in jedem Waschschritt derart durchzuführen, dass die gewaschenen Nitratester frei von Säuren und wasserlöslichen und aciden Reststoffen aus dem oxidativen Abbau des zu nitrierenden Ausgangsalkohols und/oder des Produkts sind, damit das auf diese Weise behandelte Produkt die jeweiligen Anforderungen an seine Stabilität, andere sicherheitsrelevante Anforderungen und sonstige Produktspezifikationen erfüllt und damit durch weitere Reduzierung der Mengen an Nitratester in der Anlage die Sicherheit in der Anlage steigt und damit schließlich der Aufwand und die Kosten dafür deutlich geringer sind als bei den bisher genutzten Verfahren und Vorrichtungen des Standes der Technik.

Die zuvor geschilderte Aufgabenstellung wird erfindungsgemäß durch ein Verfahren nach Anspruch 1 gelöst; weitere, vorteilhafte Weiterbildungen und Ausgestaltungen des erfindungsgemäßen Verfahrens sind Gegenstand der diesbezüglichen Unteransprüche.

Weiterer Gegenstand der vorliegenden Erfindung ist die Verwendung einer Vorrichtung bzw. eine Anlage gemäß dem betreffenden unabhängigen Patentanspruch (Nebenanspruch); weitere vorteilhafte Weiterbildungen und Ausgestaltungen dieses Erfindungsaspekts sind Gegenstand der diesbezüglichen Unteransprüche.

Darüber hinaus ist Gegenstand der vorliegenden Erfindung die Verwendung einer Produktionsanlage gemäß nach dem betreffenden unabhängigen Patentanspruch (Nebenanspruch); weitere vorteilhafte Weiterbildungen und Ausgestaltungen dieses Erfindungsaspekts sind Gegenstand der diesbezüglichen Unteransprüche.

Es versteht sich von selbst, dass Ausgestaltungen, Ausführungsformen, Vorteile oder dergleichen, welche nachfolgend - zu Zwecken der Vermeidung von unnötigen Wiederholungen - nur zu einem Erfindungsaspekt ausgeführt werden, selbstverständlich auch in Bezug auf die übrigen Erfindungsaspekte entsprechend gelten.

Weiterhin versteht es sich von selbst, dass bei nachfolgenden Angaben von Werten, Zahlen und Bereichen die diesbezüglichen Werte-, Zahlen- und Bereichsangaben nicht beschränkend zu verstehen sind; es versteht sich für den Fachmann zudem von selbst, dass einzelfallbedingt oder anwendungsbezogen von den angegebenen Bereichen bzw. Angaben abgewichen werden kann, ohne dass der Rahmen der vorliegenden Erfindung verlassen ist.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungsmethoden ermittelt bzw. bestimmt werden können.

Dies vorausgeschickt, wird im Folgenden die vorliegende Erfindung näher beschrieben.

Gegenstand der vorliegenden Erfindung - gemäß einem **ersten** Aspekt der vorliegenden Erfindung - ist somit ein Verfahren zur Entfernung von bei der Herstellung von aliphatischen oder cycloaliphatischen Salpetersäureestern (Nitratestern bzw. Nitroestern) anfallenden Verunreinigungen, insbesondere ein Verfahren zur Entfernung von Verunreinigungen aus bei der Nitrierung von nitrierbaren ein- oder mehrwertigen aliphatischen oder cycloaliphatischen Alkoholen nach Abtrennung der Nitrierendsäure anfallenden Rohsalpetersäureestern, durch Behandlung mit mindestens einem Waschmedium, wobei das Verfahren die folgenden Verfahrensschritte umfasst:
(a) zunächst werden die Rohsalpetersäureester (d. h. also die nach Abtrennung der Nitrierendsäure anfallenden nitrierten Rohprodukte) mit einem Waschmedium in Kontakt gebracht, wobei die Rohsalpetersäureester und das Waschmedium derart ineinander verteilt werden, dass eine Emulsion resultiert (d. h. mit anderen Worten wird in diesem ersten Verfahrensschritt (a) eine Emulsion bzw. Dispersion von nitrierten Rohprodukten einerseits und Waschmedium andererseits hergestellt); und
(b) nachfolgend wird die resultierende Emulsion in einen Rohrreaktor einspeist, wobei der Rohrreaktor mit Mischelementen, insbesondere zum Eintrag von zusätzlicher Mischenergie, ausgestattet ist, so dass während des Durchtritts der Emulsion durch den Rohrreaktor die in den Rohsalpetersäureestern anfänglich vorhandenen Verunreinigungen zumindest teilweise entfernt werden und/oder so dass während des Durchtritts der Emulsion durch den Rohrreaktor die in den Rohsalpetersäureestern anfänglich vorhandenen Verunreinigungen zumindest teilweise in das Waschmedium überführt und/oder hierdurch neutralisiert werden.

Das erfindungsgemäße Verfahren eignet sich somit in hervorragender Weise zur Aufreinigung von bei der Nitrierung von nitrierbaren ein- oder mehrwertigen aliphatischen Alkoholen - nach Abtrennung der Nitrierendsäure - anfallenden nitrierten Rohprodukten (d. h. also mit anderen Worten zur Aufreinigung der resultierenden Rohsalpetersäureester).

Das Prinzip des erfindungsgemäßen Verfahrens besteht also unter anderem darin, die aus der Nitrierung stammenden, noch signifikante Mengen an Verunreinigungen enthaltenden rohen Nitratester - nach Abtrennung der Nitrierendsäure (z. B. in einem Scheider) - zunächst mit einem Waschmedium in Kontakt zu bringen und die Mischung aus aufzureinigendem Nitratester und Waschmedium in eine Emulsion bzw. Dispersion ineinander zu überführen und nachfolgend die resultierende Emulsion bzw. Dispersion in einen Rohrreaktor einzuspeisen, damit die in den aufzureinigenden Nitratester anfänglich vorhandenen Verunreinigungen in das Waschmedium überführt bzw. hierdurch neutralisiert werden und auf diese Weise ein aufgereinigter Nitratester entsteht.

Typische zu entfernende Verunreinigungen umfassen beispielsweise nicht umgesetzte Edukte (d. h. insbesondere nicht umgesetzte ein- oder mehrwertige aliphatische oder cycloaliphatische Ausgangsalkohole und nicht umgesetzte Ausgangsmineralsäuren, vor allem Salpetersäure und gegebenenfalls Schwefelsäure, sofern als Edukt eingesetzt) und Reaktionsnebenprodukte (insbesondere Umsetzungsprodukte der Salpetersäure, wie z. B. Salpetrige Säure, Nitrose etc., und Reaktionsnebenprodukte der Ausgangsalkohole, wie Oxidationsprodukte, wie z. B. Carbonsäuren, Aldehyde, Ketone, Abbau- oder Spaltprodukte, Kohlendioxid etc.).

Wie die Anmelderin in vollkommen überraschender Weise herausgefunden hat, führt die Verwendung eines Rohrreaktors mit zusätzlichen Mischelementen - in Kombination mit einer vorgeschalteten Dispergier- bzw. Emulgiereinrichtung - dazu, dass eine besonders gute Durchmischung und besonders innige und feine Verteilung von Waschmedium einerseits und aufzureinigenden Nitratestern oder deren Lösungen andererseits erreicht werden kann, so dass auf diese Weise die Verunreinigungen sozusagen in einem einzigen Verfahrensschritt (nämlich im Rahmen der Rohrreaktorbehandlung) vollständig bzw. zumindest im Wesentlichen vollständig entfernt werden können.

Im Unterschied zum Stand der Technik werden also weitergehende komplexe Verfahrensschritte zur Aufreinigung des rohen Nitroesters in effizienter Weise vermieden, ohne dass Qualitätseinbußen bei der Aufreinigung des rohen Nitratestern hinzunehmen wären. Auch werden durch das erfindungsgemäße Verfahren die erforderlichen Sicherheitsstandards gewährleistet.

Überraschenderweise gewährleistet der erfindungsgemäß für die Behandlung des rohen Nitratesters mit dem Waschmedium eingesetzte Rohrreaktor mit den zusätzlichen Mischelementen - in Kombination mit der vorgeschalteten Dispergiereinrichtung zur Erzeugung der Ausgangsemulsion aus Waschmedium und Rohnitroester - eine derart innige und feine Verteilung von rohem Nitroester einerseits und Waschmedium andererseits, dass im Rahmen der Rohrreaktorbehandlung gemäß Verfahrensschritt (b) sämtliche bzw. zumindest im Wesentlichen sämtliche Verunreinigungen in das Waschmedium überführt bzw. hierdurch neutralisiert werden, so dass sie anschließend (d. h. nach Abschluss des Verfahrensschritts (b) zusammen mit dem Waschmedium von dem dann aufgereinigtem Nitratester abgetrennt werden können).

Es hat sich nämlich in überraschender Weise gezeigt, dass es im Rahmen der vorliegenden Erfindung möglich ist, eine Wäsche von Nitratestern erfolgreich quasi einstufig - auch bei hoher Belastung mit Verunreinigungen, wie Nitriersäure, Salpetersäure etc. - durchzuführen, und zwar durch eine einfache und kostengünstige Kombination von Strahlpumpen (Injektoren) oder Strahlmischern (Jet-Mixern bzw. *Jet-Mixing-Devices*), aber auch anderen Dispergierorganen, in Verbindung mit zusätzlichen Einrichtungen, wie statischen Mischern, Blenden etc., in dem nachgeschalteten Rohrreaktor, was es gestattet, nicht nur eine genau definierte Mischenergie in das Gemisch der nicht miteinander mischbaren Phasen einzutragen, sondern auch eine Verweilzeit sicherzustellen, welche es erlaubt, auch langsame Folgereaktionen möglichst quantitativ ablaufen zu lassen. Die dadurch herstellbaren Emulsionen aus der zu reinigenden Organphase im Waschmedium (O/W-Typ) oder des Waschmediums in der Organphase (W/O-Typ) liefern die für eine effektiven und optimalen Massentransfer benötigten Grenzfläche zwischen zu waschendem Nitratester und Waschmedium.

Eine Besonderheit der vorliegenden Erfindung ist auch darin zu sehen, dass der Rohrreaktor mit Mischelementen, insbesondere statischen Mischelementen, zum Eintrag von zusätzlicher Mischenergie, ausgestattet ist (wobei die Mischelemente beispielsweise als Bleche, insbesondere Prall- oder Umlenkbleche, als Blenden, als statische Mischer oder als Stromteiler ausgebildet sein können). Dies führt dazu, dass die ursprünglich vorhandene Tropfengrößenverteilung nicht nur aufrechterhalten wird, sondern sich derart einstellt, dass während des Durchtritts der Emulsion durch den Rohrreaktor die in den nitrierten Rohprodukten anfänglich vorhandenen Verunreinigungen vollständig in das Waschmedium überführt und/oder hierdurch neutralisiert werden.

Was den aufzureinigenden aliphatischen Salpetersäureester (Nitratester) anbelangt, so kann dieser aus beliebigen Salpetersäureestern ein- oder mehrwertiger aliphatischer Alkohole ausgewählt sein. Insbesondere kann der aufzureinigende Salpetersäureester ausgewählt sein aus (i) Salpetersäureestern von aliphatischen oder cycloaliphatischen Monoalkoholen, insbesondere von linearen oder verzweigten aliphatischen oder cycloaliphatischen Monoalkoholen, insbesondere von Hexanolen, Heptanolen, Octanolen, Ethylhexylalkohol und Cyclohexanol; (ii) Salpetersäureestern von zweiwertigen aliphatischen Alkoholen, insbesondere von Ethylenglykol und Propylenglykol und deren Oligomeren und Polymeren, wie Ethylen- oder Propylendiglykol, Ethylen- oder Propylentriglykol und Polyethylen- oder Polypropylenglykol; (iii) Salpetersäureestern von dreiwertigen aliphatischen Alkoholen, insbesondere von Trimethylolethan und Glycerin; (iv) Salpetersäureestern von vier- oder mehrwertigen aliphatischen Alkoholen, insbesondere von Pentaerythrit und Dipentaerythrit.

Wie zuvor ausgeführt, ist der in Verfahrensschritt (b) eingesetzte Rohrreaktor mit Mischelementen zum Eintrag von zusätzlicher Mischenergie ausgestattet. Auf diese Weise kann die ursprünglich vorhandene Tropfengrößenverteilung nicht nur aufrechterhalten bleiben, sondern vielmehr derart eingestellt werden, dass während des Durchtritts der Emulsion durch den Rohrreaktor die in den nitrierten Rohprodukten anfänglich vorhandenen Verunreinigungen in das Waschmedium überführt bzw. hierdurch neutralisiert werden.

Insbesondere sind die in dem Rohrreaktor vorhandenen Mischelemente statische Elemente, d. h. insbesondere sind die Mischelemente als statische Mischelemente ausgebildet.

Gemäß einer besonderen Ausführungsform können die Mischelemente, insbesondere die statischen Mischelemente, innenseitig im Rohrreaktor fixiert sein, insbesondere mit den Innenwandungen des Rohrreaktors verbunden sein, vorzugsweise dauerhaft oder aber lösbar verbunden sein.

Gemäß einer weiteren besonderen, alternativen Ausführungsform können die Mischelemente als Einschubelemente ausgebildet sein. Dabei können die Einschubelemente bedarfsweise in Abhängigkeit von der Anzahl und/oder Position in den Rohrreaktor eingefügt werden bzw. in dem Rohrreaktor positioniert werden.

Insbesondere können die Mischelemente als Bleche, insbesondere Prall- oder Umlenkbleche, als Blenden, als statische Mischer oder als Stromteiler ausgebildet sein.

Gemäß einer weiteren Ausführungsform sind 1 bis 15 Mischelemente, insbesondere 2 bis 15 Mischelemente, bevorzugt 2 bis 10 Mischelemente, besonders bevorzugt 2 bis 5 Mischelemente, im Rohrreaktor angeordnet. Dies führt zu einer besonders guten Emulgierung von zu reinigenden Rohrprodukten und Waschmedium.

In diesem Zusammenhang ist es weiterhin vorteilhaft, wenn durch die Mischelemente im Rohrreaktor eine ausreichende Mischenergie zur Durchmischung von Rohrprodukten und Waschmedium bereitgestellt wird. In diesem Zusammenhang ist es insbesondere vorteilhaft, wenn insgesamt im Rohrreaktor eine Mischenergie (d. h. volumenbezogene Mischenergie) von 20 bis 1000 Joule/Liter, bevorzugt 25 bis 500 Joule/Liter, besonders bevorzugt 30 bis 200 Joule/Liter, eingetragen wird.

In diesem Zusammenhang sollte Druckabfall pro Mischelement 0,2 bar bis 3,0 bar, bevorzugt 0,3 bis 1,5 bar, besonders bevorzugt 0,3 bis 0,8 bar, betragen.

Als Mischelemente besonders geeignet sind statische Mischelemente, insbesondere in Form sogenannter statischer Mischer oder Statikmischer. Hierbei handelt es sich um Vorrichtungen zum Mischen von Fluiden, bei welchen allein die Strömungsbewegung die Vermischung bewirkt und welche nicht über bewegte Elemente verfügen. Sie bestehen aus strömungsbeinflussenden Elementen in einem Rohr; diese teilen abwechselnd den Stoffstrom auf und führen ihn dann wieder zusammen, wodurch die Vermischung erreicht wird.

Erfindungsgemäß ist es also vorgesehen, um die Koaleszenz der Waschemulsion nach kurzer Zeit und damit eine nicht vollständige Extraktion der aus dem zu reinigenden Nitratester zu entfernenden Verunreinigungen zu verhindern, dass der Rohrreaktor zur Durchführung von Verfahrensschritt (b) mit Mischelementen, insbesondere zum Eintrag von zusätzlicher Mischenergie, ausgestattet ist; auf diese Weise können besonders gute Reinigungsergebnisse erzielt werden, da durch die zusätzlichen Mischelemente eine noch weiter verbesserte, besonders innige Verteilung von Waschmedium einerseits und aufzureinigendem Nitratester andererseits erreicht und über die gesamte Länge des Rohrreaktors aufrechterhalten werden kann. Bei den zusätzlichen Mischelementen, bevorzugt über den Rohrreaktor verteilt angeordnet, kann es sich insbesondere um Bleche, insbesondere Prall- oder Umlenkbleche, Blenden, statische Mischer, Stromteiler oder dergleichen handeln, um die Emulsion vom Typ O/W oder W/O aufrechtzuerhalten. Erfindungsgemäß ist es bevorzugt, wenn 1 bis 15 Mischelemente, insbesondere 2 bis 15 Mischelemente, bevorzugt 2 bis 10 Mischelemente, besonders bevorzugt 2 bis 5 Mischelemente, in dem Rohrreaktor vorliegen; diese Mischelemente können über die gesamte Länge des Rohrreaktors verteilt sein. Bevorzugt können die Mischelemente als Einschubelement in den Rohrreaktor nach Bedarf eingefügt werden.

Was die Herstellung der Emulsion bzw. Dispersion in Verfahrensschritt (a) anbelangt, so erfolgt diese im Allgemeinen mittels einer geeigneten Dispergier- bzw. Emulgiereinrichtung, insbesondere mittels eines geeigneten Mischorgans.

Im Rahmen der vorliegenden Erfindung kann als Dispergier- bzw. Emulgiereinrichtung, insbesondere als Mischorgan, beispielsweise ein Strahlmischer (Jet-Mixer) oder eine Pumpe, insbesondere eine Strahlpumpe (Injektor), eingesetzt werden.

Eine Strahlpumpe (Injektor) ist eine Pumpe, bei welcher die Pumpwirkung durch einen Fluidstrahl ("Treibmedium"), erzeugt wird, welcher durch Impulsaustausch ein anderes Medium ("Saugmedium") ansaugt, beschleunigt und verdichtet/fördert, sofern es unter ausreichendem Druck steht. Als Injektor wird üblicherweise eine Strahlpumpe bezeichnet, welche einen Überdruck erzeugt, welcher also eine vorwiegend verdichtende Wirkung hat.

Im Rahmen der vorliegenden Erfindung kann die Dispergier- bzw. Emulgiereinrichtung, insbesondere das Mischorgan, insbesondere einen Treibstrahl (Zentralstrahl) und einen den Treibstrahl umgebenden Ringstrahl erzeugen. Dabei wird bevorzugt der Treibstrahl durch das Waschmedium und der Ringstrahl durch die aufzureinigenden Rohsalpetersäureester gebildet.

Gemäß einer besondere Ausführungsform kann es vorgesehen sein, dass als Dispergier- bzw. Emulgiereinrichtung, insbesondere als Mischorgan, ein Strahlmischer (Jet-Mixer) oder eine Strahlpumpe (Injektor) eingesetzt wird. Dabei kann insbesondere der Strahlmischer oder die Strahlpumpe einen vorzugsweise zentralen Treibstrahl und einen den Treibstrahl umgebendes Medium, insbesondere in Form eines Ringstrahls, erzeugen, wobei bevorzugterweise der Treibstrahl durch das Waschmedium und der Ringstrahl durch die aufzureinigenden Rohsalpetersäureester gebildet werden kann (wobei grundsätzlich auch die umgekehrte Betriebsart möglich ist, d. h. aufzureinigende Rohsalpetersäureester als Treibstrahl und Waschmedium als Ringstrahl, obwohl diese Ausführungsform weniger bevorzugt ist).

Als Strahlmischer oder Strahlpumpen können grundsätzlich alle Arten von Strahlmischern oder Strahlpumpen eingesetzt werden, welche es gestatten, mittels des zentralen Treibstrahls als Freistrahl, welcher grundsätzlich entweder aus dem Waschmedium oder dem zu waschenden Nitratester bestehen kann, mit hoher Relativgeschwindigkeit den zu waschenden Nitratester oder das Waschmedium derart einzudüsen, dass entweder der zu waschende Nitratester im Waschmedium oder das Waschmedium im zu waschenden Nitratester als Emulsion mit großer Grenzfläche verteilt wird. Vorrichtungen dieser Art sind beispielsweise in Ullmann's Encyclopedia of Industrial Chemistry, 2003, 5. Ed., Vol. B4, Seiten 87/88 und 565 bis 571, oder aber in Perry's Chemical Engineers' Handbook, McGraw-Hill Book Company, 1984, 6. Auflage, Seiten 5-21 bis 5-23, oder aber in der deutschen Offenlegungsschrift DE 2 151 206 beschrieben.

Wie zuvor ausgeführt, kann dabei der (zentrale) Treibstrahl im Strahlmischer das Waschmedium und das umgebende Medium der aufzureinigende rohe Nitratester sein; alternativ kann aber auch der (zentrale) Treibstrahl durch das aufzureinigende nitrierte Rohprodukt und das den (zentralen) Treibstrahl umgebende Medium durch das Waschmedium gebildet sein. Beide alternativen Ausführungsformen führen zu dem gewünschten Ergebnis. Erfindungsgemäß bevorzugt ist es jedoch, wenn der (zentrale) Treibstrahl im Strahlmischer das Waschmedium und das umgebende Medium der aufzureinigende rohe Nitratester ist

Besonders gute Ergebnisse im Hinblick auf die Aufreinigung der aufzureinigenden Rohprodukte werden erhalten, wenn die Dispergier- bzw. Emulgiereinrichtung, insbesondere das Mischorgan, einen Treibstrahl (Zentralstrahl) und einen den Treibstrahl umgebenden Ringstrahl erzeugt (wobei bevorzugt der Treibstrahl durch das Waschmedium und der Ringstrahl durch die aufzureinigenden Rohsalpetersäureester gebildet werden), wobei das Verhältnis der Geschwindigkeiten zwischen dem Treibstrahl einerseits und dem den Treibstrahl umgebenden Ringstrahl andererseits im Bereich von 1 : 6 bis 35 : 1, bevorzugt im Bereich von 1 : 2 bis 25 : 1, besonders bevorzugt im Bereich von 1 : 1 bis 12 : 1, eingestellt wird. Auf diese Weise wird eine besonders innige und feine Verteilung von Waschmedium einerseits und aufzureinigendem Rohprodukt andererseits und folglich eine besonders effiziente Aufreinigung erzielt.

Die Fließgeschwindigkeit der Emulsion bzw. Dispersion aus Waschmedium und Rohprodukten im Rohrreaktor (d. h. nach der Dispergiereinrichtung) sollte im Bereich von 0,1 bis 15,0 m/s, bevorzugt 0,5 bis 10 m/s eingestellt werden.

Gemäß einer Ausführungsform der vorliegenden Erfindung kann es vorgesehen sein, dass die im Verfahrensschritt (a) eingesetzte Dispergier- bzw. Emulgiereinrichtung, insbesondere das Mischorgan, dem Rohrreaktor vorgeschaltet, insbesondere unmittelbar vorgeschaltet, ist. Gemäß einer besonderen Ausgestaltung dieser Ausführungsform kann es vorgesehen sein, dass die Dispergier- bzw. Emulgiereinrichtung, insbesondere das Mischorgan, in den Rohrreaktor übergeht.

Gleichermaßen ist es jedoch auch möglich, dass die Dispergier- bzw. Emulgiereinrichtung, insbesondere das Mischorgan, in den Rohrreaktor integriert ist bzw. Bestandteil des Rohrreaktors ist. Zu diesem Zweck kann beispielsweise die Dispergier- bzw. Emulgiereinrichtung in dem oberen bzw. stromaufwärts befindlichen Teil des Rohrreaktors angeordnet sein. Eine solche Ausführungsform ist insbesondere dann möglich, wenn die Dispergiereinrichtung, insbesondere das Mischorgan, als Strahlmischer (Jet-Mixer) oder als Strahlpumpe (Injektor) ausgebildet ist.

Was die Verweilzeit der Emulsion von Waschmedium einerseits und rohem Nitratester andererseits im Rohrreaktor (Waschapparat) im Rahmen von Verfahrensschritt (b) anbelangt, so kann diese in weiten Bereichen variieren. Besonders bevorzugt ist es, wenn die Verweilzeit im Rohrreaktor 0,05 bis 130 Sekunden, bevorzugt 0,1 bis 70 Sekunden, besonders bevorzugt 1 bis 35 Sekunden, beträgt. Auf diese Weise werden besonders gute Waschergebnisse erzielt, da zum einen eine ausreichende Mindestverweilzeit, andererseits jedoch auch ein ökonomischer Durchsatz gewährleistet wird.

Im Rahmen der Aufreinigung sind auch das Masse- und Phasenverhältnis zwischen aufzureinigenden nitrierten Rohprodukten einerseits und Waschmedium andererseits von Bedeutung, welche jeweils in weiten Bereichen variieren können.

Besonders gute Ergebnisse werden erhalten, wenn das Masseverhältnis zwischen aufzureinigenden Rohsalpetersäureestern einerseits und Waschmedium, insbesondere frisch zugesetztem Waschmedium, andererseits im Bereich von 250 : 1 bis 1 : 20, bevorzugt im Bereich von 120 : 1 bis 1 : 8, besonders bevorzugt im Bereich von 12 : 1 bis 1 : 3, eingestellt wird.

Gleichermaßen werden besonders gute Ergebnisse dann erhalten, wenn das Masseverhältnis zwischen aufzureinigenden Rohsalpetersäureestern und Waschmedium im Rohrreaktor und/oder Waschapparat im Bereich von 30 : 1 bis 1 : 6, bevorzugt im Bereich von 15 : 1 bis 1 : 5, besonders bevorzugt im Bereich von 6 : 1 bis 1 : 3, eingestellt wird.

Die Einstellung des Masse- bzw. Phasenverhältnisses wird durch eine Kreisführung des Waschmediums nach Phasentrennung erzielt. Dies gewährleistet einerseits eine optimale Austauschfläche zwischen Organphase und Waschmedium und andererseits eine möglichst kurze Zeit für die Phasentrennung im Phasentrennapparat.

Die Wäsche der rohen Nitratester kann üblicherweise als flüssig/flüssig-Wäsche in reiner Form, aber auch im Gemisch mit einem zusätzlichen inerten Lösungsmittel durchgeführt werden. Diese zusätzlichen Lösungsmittel können vor Weiterverwendung entweder abgetrennt werden oder als Gemisch Lösungsmittel/Nitratester direkt weiterverwendet werden.

Das erfindungsgemäß eingesetzte Waschmedium ist unter Verfahrensbedingungen, insbesondere bei Temperaturen ab 5° C, vorzugsweise bei Temperaturen ab 25 °C, und Atmosphärendruck flüssig ausgebildet. Erfindungsgemäß bevorzugt ist es, wenn das Waschmedium wässrig basiert ist, vorzugsweise Wasser ist.

Je nach Massen- bzw. Phasenverhältnis im Rohrreaktor (Waschapparat) wird der zu waschenden Nitratester im Waschmedium als Öl-in-Wasser-Emulsion (O/W-Emulsion) oder aber das Waschmedium im zu waschenden Nitratester als Wasserin-Öl-Emulsion (W/O-Emulsion) dispergiert.

Gemäß einer bevorzugten Ausführungsform kann das erfindungsgemäße Verfahren kontinuierlich durchgeführt werden (obwohl grundsätzlich auch eine diskontinuierliche Verfahrensweise möglich ist). Bei großtechnischer Durchführung ist eine kontinuierliche Durchführung in jedem Fall bevorzugt.

Das erfindungsgemäße Verfahren kann grundsätzlich zur Durchführung einer sauren Wäsche und/oder einer basischen Wäsche und/oder einer neutralen Wäsche der aufzureinigenden Rohsalpetersäureester eingesetzt werden.

Mit anderen Worten eignet sich das erfindungsgemäße Verfahren grundsätzlich zur Durchführung der sauren Wäsche und/oder der basischen Wäsche und/oder der neutralen Wäsche der nitrierten Rohprodukte bzw. der Rohsalpetersäureester. Das erfindungsgemäße Verfahren kann also in allen drei vorgenannten Waschschritten zum Einsatz kommen. Gleichermaßen ist es aber auch möglich, dass das erfindungsgemäße Verfahren nur für ein oder zwei Waschstufen verwendet wird, beispielsweise nur für die saure Wäsche oder aber nur für die basische Wäsche oder aber nur für die neutrale Wäsche. So kann z. B. der für Nitratester typische Emulsionstransport von Nitratestern zwischen den einzelnen Verfahrensstufen einer Produktionsanlage und/oder zur Weiterverarbeitung nach der Wäsche entsprechend dem erfindungsgemäßen Verfahren ausgebildet werden. In dieser Hinsicht ist das erfindungsgemäße Verfahren flexibel einsetzbar.

Gemäß einer besonderen Ausführungsform der vorliegenden Erfindung kann das erfindungsgemäße Verfahren, insbesondere die Verfahrensschritte (a) und (b), wiederholt durchgeführt werden bzw. mehrfach ausgeführt werden. Dabei werden die nach jeweils einem Verfahrensdurchlauf resultierenden (teil)aufgereinigten Rohsalpetersäureester erneut in den nachfolgenden Verfahrensdurchlauf eingespeist.

Bei dieser besonderen Ausführungsform kann es vorgesehen sein, dass mindestens ein Verfahrensdurchlauf als basische Wäsche und/oder mit einem basischen Waschmedium durchgeführt wird und mindestens ein hierzu nachgeschalteter Verfahrensdurchlauf als neutrale Wäsche und/oder mit einem neutralen Waschmedium durchgeführt wird.

Alternativ kann es aber auch vorgesehen sein, dass zwei, vorzugsweise drei nacheinandergeschaltete Verfahrensdurchläufe durchgeführt werden, welche gegebenenfalls eine erste Wäsche mit einem sauren Waschmedium ("saure Wäsche"), hierzu nachgeschaltet eine zweite Wäsche mit einem basischen Waschmedium ("basische Wäsche") und wiederum hierzu nachgeschaltet eine dritte Wäsche mit einem neutralen Waschmedium ("neutrale Wäsche") umfassen.

Die Effizienz des Waschmediums kann noch dadurch gesteigert werden, dass dem Waschmedium mindestens eine Base, wie nachfolgend noch beschrieben, zugesetzt wird.

Mit anderen Worten kann es erfindungsgemäß vorgesehen sein, dass dem Waschmedium, insbesondere zur Durchführung einer basischen Wäsche, mindestens eine Base zugesetzt wird. Diese Base kann insbesondere ausgewählt sein aus der Gruppe von anorganischen Hydroxiden, Carbonaten, Hydrogencarbonaten und Ammoniak sowie deren Mischungen oder Kombinationen.

Die bei einer alkalischen Wäsche eingesetzte Menge an Alkali sollte so hoch sein, dass nicht nur alle Säuren quantitativ zu ihren Salzen umgesetzt werden können, sondern es sollte ein Überschuss an Base eingesetzt werden, damit der pH-Wert in der Waschlauge so hoch ist, dass auch schwache Säuren quantitativ ausgewaschen werden können. Insbesondere ist es in diesem Zusammenhang vorteilhaft, wenn der Gehalt an Base im Waschmedium 0,01 bis 0,4 mol/l, bevorzugt 0,02 bis 0,2 mol/l, beträgt. Insbesondere sollte der Gehalt an Base im Waschmedium mindestens das Doppelte der für die Neutralisation benötigten Alkalimenge aller als Verunreinigungen enthaltener mit Basen Salze bildenden Stoffe betragen.

Wie bereits zuvor ausgeführt, sind die aufzureinigenden Rohsalpetersäureester unter Verfahrensbedingungen flüssig ausgebildet. Gegebenenfalls können die aufzureinigenden Rohsalpetersäureester in einem inerten Lösungsmittel gelöst vorliegen, so z. B. in halogenhaltigen Kohlenwasserstoffen, insbesondere Methylenchlorid, Dichlorethan etc., oder in anderen Lösungsmitteln, welche die Weiterverarbeitung nicht stören oder bei der Weiterverarbeitung eingesetzt werden.

Dem Verfahrensschritt (b) kann sich dann eine Abtrennung der von den Verunreinigungen befreiten Salpetersäureestern vom Waschmedium, vorzugsweise in einer Phasentrenneinrichtung (Scheider), anschließen.

Als Phasentrenneinrichtung bzw. Phasentrennapparat können alle Arten von statischen Scheidern zum Einsatz kommen, aber auch dynamische Scheider, wie Zentrifugalseparatoren. Die Scheidezeit der Emulsion Nitratester/Waschmedium hängt, neben der Dichtedifferenz zwischen den beiden Phasen, vom Emulsionstyp (W/O oder O/W) und der eingetragenen Mischenergie auch noch von dem Überschuss an Base im Waschmedium, der nicht zur Neutralisation benötigt wird, ab. Bei Eintrag gleicher Mischenergie sinkt die Scheidezeit deutlich mit zunehmender Basekonzentration im Waschmedium. Zur Beschleunigung der Phasentrennung können aber auch oberflächenaktive Mittel oder auch mechanische Trennhilfen, wie Packungen, Trennbleche etc., eingesetzt werden. Auch durch einen auf den Nitratester und Emulsionstyp abgestimmten Abstand zwischen den einzelnen Mischelementen kann die Phasentrennung beschleunigt werden.

Gemäß einer vorteilhaften Ausführungsform des erfindungsgemäßen Verfahrens ist es vorgesehen, dass das Waschmedium, insbesondere nach Abtrennung der von den Verunreinigungen befreiten nitrierten Salpetersäureester vom Waschmedium, rezykliert wird. Auf diese Weise wird eine effiziente Wäsche ermöglicht und die Menge an Waschmedium auf ein Minimum reduziert.

Zur Entfernung von Verunreinigungen mit hohen Verteilungskoeffizienten zugunsten des zu waschenden Nitratesters, hohen Massentransferwiderständen in der Organphase und langsamen Weiterreaktionen der extrahierten Verunreinigungen im Waschmedium, wie z. B. Nitrose oder Stickstoffdioxid, sollte die Verweilzeit im nachfolgenden Reaktor an diese Verhältnisse angepasst werden. Gemäß einer besonderen Ausführung des erfindungsgemäßen Verfahrens kann dies z. B. durch eine Kombination der vorstehend beschriebenen Einrichtungen zur Erzeugung einer optimalen Waschemulsion zusammen z. B. mit Rührkesseln erreicht werden, um die notwendige Verweilzeit für den Phasentransfer und die nachfolgende Umsetzung sicherzustellen.

Die am Ende der Mischstrecke vorliegende Dispersion/Emulsion kann in einem Phasentrennapparat (Scheider bzw. Settler) wieder in die einzelnen Phasen getrennt werden. Das Waschmedium mit den darin enthaltenen Verunreinigungen kann entweder als Abwasser einer Abwasserbehandlung zugeführt oder im Gegenstrom in die vorgeschaltete Waschstufe eingebracht werden.

Der gewaschene Nitratester kann entweder in die nachfolgende Waschstufe eingespeist oder am Ende der Wäsche direkt zur Weiterverarbeitung oder in ein Zwischenlager transferiert werden.

Gegebenenfalls können nach der Wäsche bzw. nach Abtrennung des Waschmediums (z. B. nach einer Trennung der Waschemulsion in einem statischen Scheider oder durch einen Zentrifugalseparator) Spuren an suspendiertem und gelöstem Wasser aus dem aufgereinigten Nitratester durch eine zusätzliche Trocknung aus dem aufgereinigten Nitratester entfernt werden, sofern gewünscht.

Wie zuvor geschildert, ist das erfindungsgemäße Verfahren mit einer Vielzahl von Vorteilen und Besonderheiten verbunden, von denen nachfolgend einige Vorteile und Besonderheiten - jedoch nicht abschließend und in nicht beschränkender Weise - aufgeführt werden sollen:
Insbesondere ermöglicht das erfindungsgemäße Verfahren eine effiziente Aufreinigung von aus bei der Nitrierung von nitrierbaren ein- und mehrwertiger (cyclo)aliphatischen Alkoholen nach Abtrennung der Nitrierendsäure anfallenden nitrierten Rohprodukten mit nur geringer Komplexität und großer Verfahrensökonomie wie Verfahrenseffizienz.

Der erfindungsgemäß für die Aufreinigung der rohen Nitrierprodukte zum Einsatz kommende Rohrreaktor ermöglicht die Erzeugung großer Austauschflächen für ein zweiphasiges Gemisch aus Waschmedium einerseits und rohem Nitratester andererseits, so dass auf diese Weise ein effektiver Massentransfer und eine schnelle Überführung der Verunreinigungen in das Waschmedium bzw. im Fall saurer Verbindungen eine schnelle Neutralisation gewährleistet wird.

Die erfindungsgemäße Verfahrensführung erlaubt eine schnelle und gleichsam effiziente Beseitigung der aus der Nitrierung stammenden Verunreinigungen aus den nitrierten Rohprodukten, wobei das Waschmedium nach der Behandlung der Nitratester ohne Weiteres rezykliert bzw. im Kreis gefahren werden kann.

Weiterhin wird durch den konsequenten Einsatz des erfindungsgemäßen Verfahrens die allgemeine Sicherheit beim Umgang mit den hochempfindlichen mehrfach nitrierten Nitratestern, wie NGL oder EGDN etc., weiter verbessert. Die in der Anlage vorhandenen Sprengstoffmengen werden weiter reduziert.

Der erfindungsgemäß eingesetzte Rohrreaktor mit zusätzlichen Mischelementen erlaubt nicht nur eine effiziente und innige Verteilung von Waschmedium einerseits und nitriertem (cyclo)aliphatischem Alkohol andererseits ineinander, sondern durch die gezielte Einstellung einer definierten Dispersion des zu waschenden Nitratesters im Waschmedium über die gesamte Länge des Rohrreaktors wird, unter Beachtung der zulässigen Rohrquerschnitte, eine teilweise Koaleszenz des dispergierten Nitratesters im Rohrreaktor sicher verhindert und damit die Ausbildung von Stromfäden an abgeschiedenem Nitratester, die eine Detonation übertragen könnten.

Das erfindungsgemäße Verfahren eignet sich zur Durchführung der sauren Wäsche und/oder der basischen Wäsche und/oder der neutralen Wäsche der nitrierten Rohprodukte. Das erfindungsgemäße Verfahren kann also in allen drei vorgenannten Waschschritten zum Einsatz kommen. Gleichermaßen ist es aber auch möglich, das erfindungsgemäße Verfahren nur für eine oder zwei Waschstufen zu verwenden, beispielsweise nur für die saure Wäsche oder aber nur für die basische Wäsche oder aber nur für die neutrale Wäsche. So kann z.B. der für Nitratester typische Emulsionstransport von Nitratestern zwischen den einzelnen Prozessstufen einer Produktionsanlage und/oder zur Weiterverarbeitung nach der Wäsche entsprechend dem erfindungsgemäßen Verfahren ausgebildet werden. In dieser Hinsicht ist das erfindungsgemäße Verfahren flexibel einsetzbar.

Weiterer Gegenstand der vorliegenden Erfindung - gemäß einem **zweiten** Aspekt der vorliegenden Erfindung - ist die Verwendung einer Vorrichtung (Anlage) zur Entfernung von bei der Herstellung von aliphatischen oder cycloaliphatischen Salpetersäureestern (Nitratestern) anfallenden Verunreinigungen, insbesondere zur Entfernung von Verunreinigungen aus bei der Nitrierung von nitrierbaren ein- oder mehrwertigen aliphatischen oder cycloaliphatischen Alkoholen nach Abtrennung der Nitrierendsäure anfallenden Rohsalpetersäureestern, durch Behandlung mit mindestens einem Waschmedium, wobei sich die erfindungsgemäß eingesetzte Vorrichtung insbesondere zur Durchführung eines wie zuvor geschilderten Verfahrens eignet,
wobei die Vorrichtung die folgenden Einrichtungen aufweist:
(a) mindestens eine Dispergiereinrichtung, insbesondere mindestens ein Mischorgan, zum Inkontaktbringen und Emulgieren von aufzureinigenden Rohsalpetersäureestern einerseits und Waschmedium andererseits; und,
(b) stromabwärts zur Dispergiereinrichtung angeordnet, einen Rohrreaktor zur Einspeisung der in der Dispergiereinrichtung erzeugten Emulsion von aufzureinigenden Rohsalpetersäureestern einerseits und Waschmedium andererseits, wobei der Rohrreaktor mit Mischelementen, insbesondere zum Eintrag von zusätzlicher Mischenergie, ausgestattet ist, so dass während des Durchtritts der Emulsion durch den Rohrreaktor die in den Rohsalpetersäureestern anfänglich vorhandenen Verunreinigungen zumindest teilweise entfernt werden und/oder so dass während des Durchtritts der Emulsion durch den Rohrreaktor die in den Rohsalpetersäureestern anfänglich vorhandenen Verunreinigungen zumindest teilweise in das Waschmedium überführt und/oder hierdurch neutralisiert werden;
(c) stromabwärts zum Rohrreaktor angeordnet, eine Abtrenneinrichtung, insbesondere eine Scheideeinrichtung (Scheider), zur Abtrennung der von den Verunreinigungen befreiten nitrierten Produkte vom Waschmedium angeordnet ist.

Wie zuvor im Zusammenhang mit dem erfindungsgemäßen Verfahren geschildert, kann die Dispergiereinrichtung, insbesondere das Mischorgan, als Strahlmischer (Jet-Mixer) oder Pumpe, insbesondere Strahlpumpe (Injektor), ausgebildet sein.

Wie gleichermaßen zuvor im Rahmen des erfindungsgemäßen Verfahren beschrieben, kann die Dispergiereinrichtung, insbesondere das Mischorgan, derart ausgebildet sein, dass sie einen Treibstrahl (Zentralstrahl) und einen den Treibstrahl umgebenden Ringstrahl erzeugt.

Wie gleichermaßen im Zusammenhang mit dem erfindungsgemäßen Verfahren geschildert, kann die Dispergiereinrichtung, insbesondere das Mischorgan, als Strahlmischer (Jet-Mixer) oder Strahlpumpe (Injektor) ausgebildet sein. Dabei kann der Strahlmischer oder die Strahlpumpe insbesondere derart ausgebildet sein, dass ein vorzugsweise zentraler Treibstahl und ein den Treibstrahl umgebendes Medium, insbesondere in Form eines Ringstrahls, erzeugbar ist.

Wie zuvor im Zusammenhang mit dem erfindungsgemäßen Verfahren bereits beschreiben, kann die Dispergiereinrichtung, insbesondere das Mischorgan, dem Reaktor vorgeschaltet, insbesondere unmittelbar vorgeschaltet, sein, wobei gemäß einer besonderen Ausführungsform die Dispergiereinrichtung, insbesondere das Mischorgan, in den Rohrreaktor übergehen kann.

Gemäß einer alternativen Ausführungsform kann die Dispergiereinrichtung, insbesondere das Mischorgan, in den Rohrreaktor integriert sein und/oder Bestandteil des Rohrreaktors sein. Diesbezüglich kann auf die obigen Ausführungen im Zusammenhang mit dem erfindungsgemäßen Verfahren verwiesen werden.

Wie zuvor bei der Schilderung des erfindungsgemäßen Verfahrens erläutert, ist der Rohrreaktor mit Mischelementen, insbesondere zum Eintrag von zusätzlicher Mischenergie, ausgestattet. Bezüglich weiterer Einzelheiten kann auf das erfindungsgemäße Verfahren verwiesen werden.

Im Rahmen der erfindungsgemäß eingesetzten Vorrichtung kann eine ein-, zwei- oder dreistufige Wäsche des rohen Nitrierprodukts durchgeführt werden (d. h. saure Wäsche und/oder basische Wäsche und/oder neutrale Wäsche).

Weiterhin ist es erfindungsgemäß vorgesehen, dass - stromabwärts zum Rohrreaktor angeordnet - eine Abtrenneinrichtung, insbesondere eine Scheideeinrichtung (Scheider), zur Abtrennung der von den Verunreinigungen befreiten nitrierten Produkte vom Waschmedium angeordnet ist.

Des Weiteren besteht im Rahmen der erfindungsgemäß eingesetzten Vorrichtung die Möglichkeit, dass - stromabwärts zum Rohrreaktor und stromaufwärts zur Abtrenneinrichtung (d. h. mit anderen Worten zwischen Rohrreaktor und Abtrenneinrichtung) - ein Rührkessel und/oder ein Rührreaktor angeordnet ist. Insbesondere wird auf diese Weise die Kontakt und/oder Verweilzeit zwischen nitrierten Produkten (Salpetersäureestern) einerseits und dem Waschmedium andererseits verlängert.

Für weitergehende Einzelheiten zu der erfindungsgemäß eingesetzten Vorrichtung bzw. Anlage kann zur Vermeidung unnötiger Wiederholungen auf die obigen Ausführungen zu dem erfindungsgemäßen Verfahren verwiesen werden, welche in Bezug auf die erfindungsgemäß eingesetzte Vorrichtung bzw. Anlage entsprechend gelten.

Schließlich ist weiterer Gegenstand der vorliegenden Erfindung - gemäß einem **dritten Aspekt** der vorliegenden Erfindung - die Verwendung einer Produktionsanlage zur Herstellung von Salpetersäureestern ein- oder mehrwertiger aliphatischer oder cycloaliphatischer Alkohole und/oder zur Nitrierung ein- oder mehrwertiger aliphatischer oder cycloaliphatischer Alkohole mit nachfolgender Aufreinigung der bei der Nitrierung entstehenden nitrierten Rohprodukte,
wobei die Produktionsanlage die folgenden Einheiten umfasst:
(a) eine Nitriereinheit zur Herstellung von Salpetersäureestern ein- oder mehrwertiger aliphatischer oder cycloaliphatischer Alkohole und/oder zur Nitrierung ein- oder mehrwertiger aliphatischer oder cycloaliphatischer Alkohole, insbesondere mit einem oder mehreren entsprechenden Reaktionsbehältern zur Durchführung der Nitrierreaktion(en);
(b) gegebenenfalls, in Produktionslinie stromabwärts zur Nitriereinheit angeordnet, eine Abtrenneinrichtung, insbesondere eine Scheideeinrichtung (Scheider), insbesondere zur Abtrennung der Nitrierendsäure von den nitrierten Rohprodukten (Rohsalpetersäureestern);
(c) in Produktionslinie stromabwärts zur Nitriereinheit und zur gegebenenfalls vorhandenen Abtrenneinrichtung angeordnet, eine Wascheinrichtung zur Durchführung einer Wäsche der nitrierten Rohprodukte, wobei die Wascheinrichtung umfasst:
   - mindestens eine Dispergiereinrichtung, insbesondere mindestens ein Mischorgan, zum Inkontaktbringen und Emulgieren von aufzureinigenden Rohsalpetersäureestern einerseits und Waschmedium andererseits; und,
   - stromabwärts zur Dispergiereinrichtung angeordnet, einen Rohrreaktor zur Einspeisung der in der Dispergiereinrichtung erzeugten Emulsion von aufzureinigenden Rohsalpetersäureestern einerseits und Waschmedium andererseits, wobei der Rohrreaktor mit Mischelementen, insbesondere zum Eintrag von zusätzlicher Mischenergie, ausgestattet ist, so dass während des Durchtritts der Emulsion durch den Rohrreaktor die in den Rohsalpetersäureestern anfänglich vorhandenen Verunreinigungen zumindest teilweise entfernt werden und/oder so dass während des Durchtritts der Emulsion durch den Rohrreaktor die in den Rohsalpetersäureestern anfänglich vorhandenen Verunreinigungen zumindest teilweise in das Waschmedium überführt und/oder hierdurch neutralisiert werden;
(d) gegebenenfalls, in Produktionslinie stromabwärts zur Wascheinrichtung angeordnet, einen Rührkessel, insbesondere zur Erhöhung der Kontakt- und/oder Verweilzeit zwischen Salpetersäureestern einerseits und Waschmedium andererseits;
(e) in Produktionslinie stromabwärts zur Wascheinheit und zum gegebenenfalls vorhandenen Rührkessel angeordnet, eine Abtrenneinrichtung, insbesondere eine Scheideeinrichtung (Scheider), insbesondere zur Abtrennung der von den Verunreinigungen befreiten Salpetersäureester vom Waschmedium.

Mit anderen Worten ist bei der erfindungsgemäß eingesetzten Produktionsanlage die zuvor beschriebene Vorrichtung bzw. Anlage zur Aufreinigung, d. h. zur Entfernung von Verunreinigungen, Bestandteil dieser Produktionsanlage, nämlich in Form der Wascheinheit bzw. Wascheinrichtung (c).

Wie zuvor bereits geschildert, können die Mischelemente im Rohrreaktor statische Mischelemente sein bzw. als statische Mischelemente ausgebildet werden.

Gemäß einer besonderen Ausführungsform können die Mischelemente, insbesondere die statischen Mischelemente, innenseitig im Rohrreaktor fixiert sein, insbesondere mit den Innenwandungen des Rohrreaktors verbunden dauerhaft oder lösbar verbunden sein.

Alternativ können die Mischelemente aber auch als Einschubelemente ausgebildet sein. Dabei können die Einschubelemente bedarfsweise nach Anzahl und/oder Position in den Rohrreaktor eingefügt bzw. in dem Rohrreaktor positioniert werden.

Wie zuvor geschildert, können die Mischelemente als Bleche, insbesondere Prall- oder Umlenkbleche, als Blenden, als statische Mischer oder als Stromteiler ausgebildet sein.

Insbesondere können 1 bis 15 Mischelemente, insbesondere 2 bis 15 Mischelemente, bevorzugt 2 bis 10 Mischelemente, besonders bevorzugt 2 bis 5 Mischelemente, im Rohrreaktor angeordnet sein.

Wie zuvor ausgeführt, ist es vorteilhaft, wenn durch die Mischelemente im Rohrreaktor eine Mischenergie (d. h. volumenbezogene Mischenergie) von 20 bis 1000 Joule/Liter, bevorzugt 25 bis 500 Joule/Liter, besonders bevorzugt 30 bis 200 Joule/Liter, eingetragen wird und/oder wenn der Druckabfall pro Mischelement 0,2 bar bis 3,0 bar, bevorzugt 0,3 bis 1,5 bar, besonders bevorzugt 0,3 bis 0,8 bar, beträgt.

Wie zuvor im Zusammenhang mit dem erfindungsgemäßen Verfahren und der erfindungsgemäß eingesetzten Vorrichtung bzw. Anlage bereits geschildert, kann die Dispergiereinrichtung, insbesondere das Mischorgan, als Strahlmischer (Jet-Mixer) oder Pumpe, insbesondere Strahlpumpe (Injektor), ausgebildet sein.

Weiterhin kann die Dispergiereinrichtung, insbesondere das Mischorgan, derart ausgebildet sein, dass sie einen Treibstrahl (Zentralstrahl) und einen den Treibstrahl umgebenden Ringstrahl erzeugt.

Gemäß einer besonderen Ausführungsform kann die Dispergiereinrichtung, insbesondere das Mischorgan, als Strahlmischer (Jet-Mixer) oder Strahlpumpe (Injektor) ausgebildet sein. Dabei kann der Strahlmischer oder die Strahlpumpe derart ausgebildet sein, dass ein vorzugsweise zentraler Treibstahl und ein den Treibstrahl umgebendes Medium, insbesondere in Form eines Ringstrahls, erzeugbar ist.

Wie bereits zuvor geschildert, kann die Dispergiereinrichtung, insbesondere das Mischorgan, dem Reaktor vorgeschaltet, insbesondere unmittelbar vorgeschaltet, sein. Bei dieser Ausführungsform kann die Dispergiereinrichtung, insbesondere das Mischorgan, in den Rohrreaktor übergehen.

Gemäß einer alternativen Ausführungsform kann die Dispergiereinrichtung, insbesondere das Mischorgan, aber auch in den Rohrreaktor integriert sein bzw. Bestandteil des Rohrreaktors sein.

Für weitergehende Einzelheiten zu der erfindungsgemäß eingesetzten Produktionsanlage kann auf die obigen Ausführungen zu dem erfindungsgemäßen Verfahren und zu der erfindungsgemäß eingesetzten Vorrichtung bzw. Anlage verwiesen werden, welche in Bezug auf die erfindungsgemäß eingesetzte Produktionsanlage entsprechend gelten.

Das erfindungsgemäße Verfahren und die erfindungsgemäß verwendete Vorrichtung bzw. Anlage zur Aufreinigung sowie die erfindungsgemäß verwendete Produktionsanlage zur Nitrierung sind in den beigefügten Figurendarstellungen beispielhaft und in nichtbeschränkender Weise veranschaulicht.

Weitere Vorteile, Eigenschaften, Aspekte und Merkmale der vorliegenden Erfindung ergeben sich aus der folgenden Beschreibung von in den Zeichnungen dargestellten, erfindungsgemäß bevorzugten Ausführungsformen. Es zeigt:
- Fig. 1: eine schematische Darstellung einer einstufigen Wäsche für rohe Nitratester nach dem erfindungsgemäßen Verfahren bzw. mit der erfindungsgemäß eingesetzten Vorrichtung;
- Fig. 2: eine detailliertere Darstellung einzelner Teile der Vorrichtung nach der schematischen Darstellung des erfindungsgemäßen Verfahrens gemäß Fig. 1;
- Fig. 3: eine schematische Darstellung eines Ablaufs des erfindungsgemäßen Verfahrens bzw. eine schematische Darstellung der erfindungsgemäß eingesetzten Vorrichtung bzw. Anlage gemäß einem bevorzugten Ausführungsbeispiel der Erfindung für die üblichen drei Waschstufen einer Wäsche von Nitratestern;
- Fig. 4: eine schematische Darstellung einer erfindungsgemäß eingesetzten Produktionsanlage zur Nitrierung nitrierbarer ein- und mehrwertiger Alkohole mit nachfolgender Wäsche der erhaltenen Nitratester gemäß einem bevorzugten Ausführungsbeispiel der Erfindung.

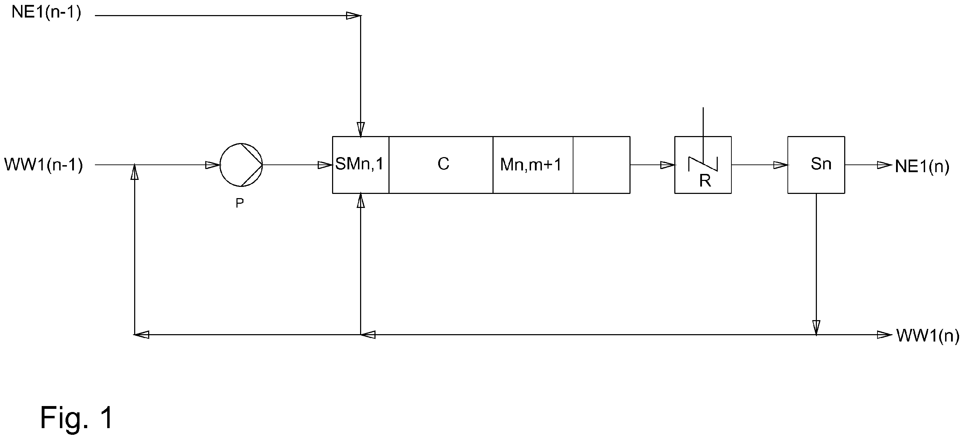

Fig. 1 zeigt - in schematischer Darstellung - eine Ausführungsform für eine Waschstufe nach dem erfindungsgemäßen Verfahren bzw. mit der erfindungsgemäß eingesetzten Vorrichtung bzw. Anlage zur Wäsche von rohen Nitratestern mit dem Waschmedium als Treibstrahl.

Der zu waschende rohe Nitratester NE1(n-1) mit n = 1-3 wird nach der Abtrennung der Nitrierendsäure in einer Wäsche WW1(n-1) mit n = 1-3 - d. h. in einer sauren Wäsche WS mit n = 1 oder nach der Entfernung aller im Nitratester noch gelösten anderen schwach aciden Stoffe aus dem oxidativen Abbau von Verunreinigungen in Gegenwart von Basen in einer alkalische Wäsche WA mit n = 2 oder in einer Neutralwäsche WN mit n=3 - in einer Mischeinrichtung SMn,1 mit n = 1-3, bevorzugt in einer Strahlpumpe (Injektor) oder einem Strahlmischer, mit dem Waschmedium WW10 (Frischwasser) bzw. WW1n mit n = 1-3, welches im dargestellten Fall als Treibstrahl dient, zusammengebracht und direkt in einen Rohrreaktor C, welcher der zusätzliche Mischelementen Mn,m+1 mit n = 1-3 und mit m = 1-15 enthält, eingebracht.

Die Waschemulsion aus dem Rohrreaktor wird direkt oder, sofern erforderlich, nach einer verlängerten Verweilzeit in einem Verweilzeitbehälter (z. B. in einem Rührkessel R etc.) in einer Scheideeinrichtung Sn mit n = 1-3 in die Phasen getrennt.

Der gewaschene Nitratester NE1n mit n = 1-3 wird entweder in die nachfolgende Waschstufe bzw. als fertig gewaschenes Produkt NE13 zur Weiterverarbeitung abgegeben. Das beladene Waschmedium WW1n mit n = 1-3 wird entweder direkt als Abwasser abgegeben oder aber als Teilstrom zur Einstellung eines definierten Phasenverhältnisses zwischen Nitratester und Waschmedium zurückgeführt. Dieser zurückgeführte Teilstrom kann entweder zusammen mit dem neu zugesetzten Waschwasser als Treibstrahl oder als Umlaufstrom direkt in den Rohrreaktor eingespeist werden.

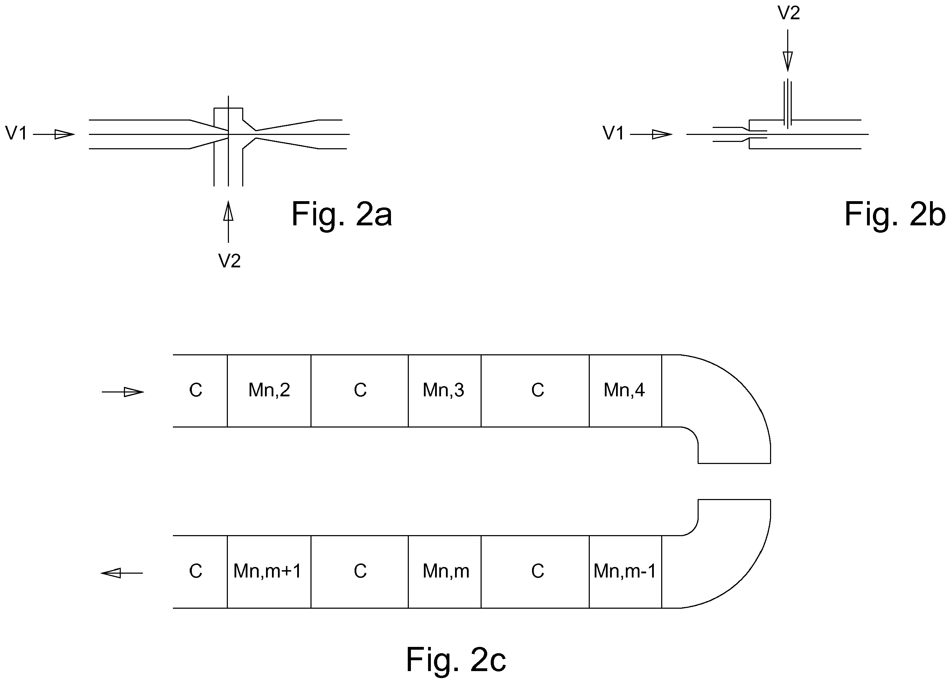

Die Figurendarstellungen gemäß Fig. 2a-2c zeigen die in der Fig. 1 schematisch dargestellte Mischeinrichtungen SMn,1 mit n = 1-3 als Strahlpumpe (Fig. 2a) und als Strahlmischer (Fig. 2b), wobei V1 für den Treibstrahl (vorzugsweise Waschmedium) und V2 für den Nitratester steht. In Fig. 2c ist zusätzlich eine mögliche Anordnung der statischen Mischelemente Mn,2, Mn,3 Mn,4, Mn,m-1, Mn,m und Mn,m+1 im Rohrreaktor, und zwar verteilt über die gesamte Länge des Rohrreaktors, dargestellt.

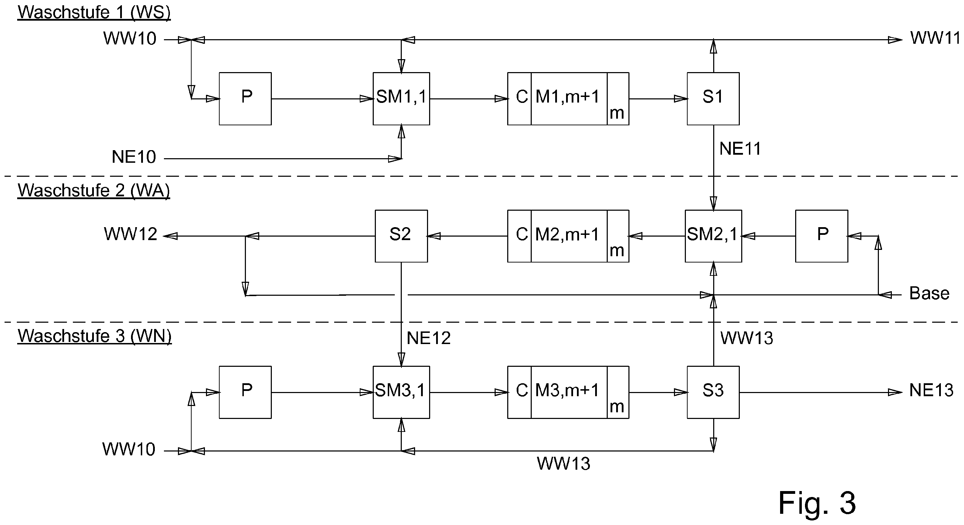

Fig. 3 zeigt ein Beispiel des erfindungsgemäßen Verfahrens in drei Schritten für die separate Entfernung der Mineralsäuren mittels saurer Wäsche (WS), für die Entfernung aller sonst noch vorhandenen schwach aciden Stoffe aus dem oxidativen Abbau von Verunreinigungen in Gegenwart von Basen im alkalischen Bereich mittels alkalischer Wäsche (WA) und für die Entfernung von Spuren mitgerissener alkalischer Waschlauge mittels Neutralwäsche (WN):
a) In Schritt 1 werden in einer einstufigen sauren Wäsche WS die im rohen Nitratester NE 10 suspendierte und gelöste Schwefel- und Salpetersäure durch Wäsche mit Frischwasser WW 10 entfernt. Das Waschwasser WW 10 und zurückgeführtes Waschwasser WW 11 als Treibstrahl werden mittels Pumpe P zusammen mit dem zu waschenden Nitratester NE 10 mit einer Strahlpumpe (Injektor) oder über einen Strahlmischer direkt in einen Rohrreaktor eingespeist, der zusätzliche Mischelemente M1,m+1 enthält. Nach Durchlauf durch den Rohrreaktor wird die gebildete Emulsion in einem Scheider S1 getrennt. Das Waschmedium wird nach Phasentrennung entweder als Abwasser WW 11 direkt abgegeben, oder aber alternativ hierzu wird eine Teilmenge zusätzlich im Kreis geführt, um ein vorgegebenes Phasenverhältnis und damit einen definierten Emulsionstyp einzustellen. Der von Mineralsäuren befreite Nitratester NA 11 wird in Waschstufe 2, die alkalische Wäsche WA, eingespeist.
b) In Schritt 2 werden in einer einstufigen alkalischen Wäsche WA alle noch gelösten Mineralsäuren und andere acide Stoffe aus dem oxidativen Abbau von Verunreinigungen, entfernt. Das Waschwasser WW 13 aus der Neutralwäsche und zurückgeführtes Waschwasser WW12 als Treibstrahl werden unter Zusatz einer Base mittels Pumpe P zusammen mit dem zu waschenden Nitratester NE 11 aus der sauren Wäsche WS mit einer Strahlpumpe (Injektor) oder über einen Strahlmischer direkt in einen Rohrreaktor eingespeist, welcher zusätzliche Mischelemente M2,m+1 enthält. Nach Durchlauf durch den Rohrreaktor wird die gebildete Emulsion in einem Scheider S2 getrennt. Das Abwasser WW 12 mit einem pH größer als 7,5 wird entweder direkt abgegeben, oder aber alternativ kann eine Teilmenge im Kreis geführt werden, um ein vorgegebenes Phasenverhältnis und damit einen definierten Emulsionstyp einzustellen. Der von allen Mineralsäuren und anderen aciden Stoffen aus dem oxidativen Abbau von Verunreinigungen befreite Nitratester NA 12 wird in die Waschstufe 3, die Neutralwäsche WN, eingespeist.
c) In Schritt 3 werden in einer einstufigen Neutralwäsche WN die mitgerissenen Spuren an Waschmedium aus der alkalischen Wäsche entfernt. Das Waschwasser WW 10 und zurückgeführtes Waschwasser WW 13 als Treibstrahl werden mittels Pumpe P zusammen mit dem zu waschenden Nitratester NE 12 mit einer Strahlpumpe (Injektor) oder über einen Strahlmischer direkt in einen Rohrreaktor eingespeist, welcher zusätzliche Mischelemente M3,m+1 enthält. Nach Durchlauf durch den Rohrreaktor wird die gebildete Emulsion in einem Scheider S3 getrennt. Das Waschmedium, das die Restspuren an Alkali und Verunreinigungen enthält, wird entweder als Abwasser WW 13 direkt in die zweite Waschstufe WA eingeführt, oder aber alternativ kann eine Teilmenge zusätzlich im Kreis geführt werden, um ein vorgegebenes Phasenverhältnis und damit einen definierten Emulsionstyp einzustellen. Der von Mineralsäuren und anderen aciden Stoffen aus dem oxidativen Abbau von Verunreinigungen und Restspuren Alkali befreite Nitratester NE 13 wird direkt zur Weiterverarbeitung oder nach Abtrennung von möglicherweise vorhandenen inerten Lösungsmitteln in ein Zwischenlager abgegeben.

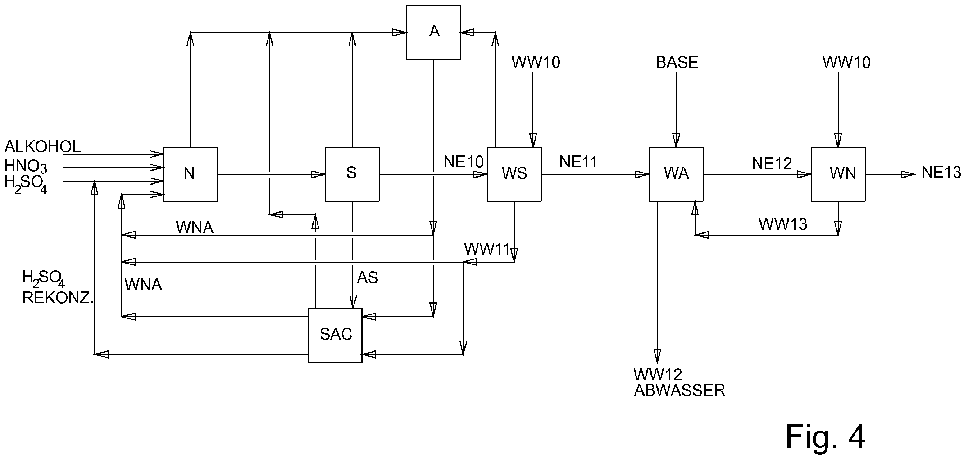

Fig. 4 zeigt ein Beispiel für eine erfindungsgemäß eingesetzte Produktionsanlage zur Herstellung von Nitratestern mit integrierter erfindungsgemäßer Wäsche der rohen Nitratester aus einer isothermen oder adiabatischen Nitrierung. Der in der Nitriereinheit N durch Umsetzung des zu nitrierenden Alkohols mit Salpetersäure in Gegenwart von Schwefelsäure gebildete rohe Nitratester NE 10 wird nach Abtrennung der Nitriersäure im Scheider S in der sauren Wäsche WS mit Wasser WW 10 in der erfindungsgemäßen Weise gewaschen. Nach Phasentrennung wird das resultierende Abwasser WW 11, welches alle ausgewaschene Schwefel- und Salpetersäure enthält, zusammen mit der aus der Abgasbehandlung der Nitrieranlage in einer Absorberanlage A erhaltenen Salpetersäure WNA entweder direkt oder nach Aufkonzentrierung in einer SAC-Anlage SAC zusammen mit der Endsäure AS aus der Nitrierung wieder in die Nitrierung zurückgeführt oder als zu behandelndes Abwasser abgegeben.

Der von den Mineralsäuren befreite Nitratester NE 11 wird in der Waschstufe 2 (d. h. alkalische Wäsche WA) in Gegenwart von Basen nach dem erfindungsgemäßen Verfahren sozusagen einstufig gewaschen. Nach Phasentrennung wird das aus der alkalischen Wäsche stammende Abwasser WW12 mit einem pH-Wert im Bereich von 7,5 bis 13, welches noch alle anderen aciden Stoffe aus dem oxidativen Abbau von Verunreinigungen enthält, vor Abgabe in einen Vorfluter einer zusätzlichen Behandlung zugeführt. Der Nitratester NA 12 aus der alkalischen Wäsche WA wird in die Neutralwäsche WN eingespeist und mit Wasser WW 10 nach dem erfindungsgemäßen Verfahren sozusagen einstufig gewaschen. Nach Phasentrennung wird das aus der Neutralwäsche WN stammende Abwasser WW 13 in die alkalische Waschstufe 2 (WA) zusammen mit Base eingespeist. Der gewaschene Nitratester NA 13 wird direkt in die Weiterverarbeitung oder in ein Zwischenlager abgegeben.

Weitere Ausgestaltungen, Abwandlungen, Variationen der vorliegenden Erfindung sind für den Fachmann beim Lesen der Beschreibung ohne Weiteres erkennbar und realisierbar, ohne dass der Rahmen der vorliegenden Erfindung verlassen wird.

Die vorliegende Erfindung wird anhand der folgenden Ausführungsbeispiele veranschaulicht, ohne jedoch die vorliegende Erfindung hierauf zu beschränken.

### Ausführungsbeispiele:

### Wäsche von Ethylhexylnitrat (EHN)

In den nachfolgenden Ausführungsbeispielen wird die Wäsche eines aus der Nitrierung hervorgegangen rohen, d. h. noch ungewaschenen und mit aus der Nitrierung stammenden Nebenprodukten und nicht umgesetzten Ausgangschemikalien verunreinigten Ethylhexylnitrats beschrieben. Zielsetzung ist es unter anderem, einen Gesamtsäuregehalt von weniger als 30 ppm, berechnet als Salpetersäure (marktübliche Produktspezifikation), zu erhalten.

Auch wenn in den nachfolgenden Ausführungsbeispielen das erfindungsgemäße Verfahren bzw. die erfindungsgemäß eingesetzte Vorrichtung mit Ethylhexylnitrat (EHN) als aufzureinigendem Nitratester veranschaulicht wird, ist das Verfahren bzw. die Vorrichtung nach der vorliegenden Erfindung keinesfalls darauf beschränkt, sondern ist auch auf beliebige andere Nitratester, z. B. Nitratestern aus der Nitrierung von Glycerin, Trimethylolpropan, Propandiol, Ethylenglykol, Di- und Triethylenglykol etc., und auf beliebige andere Basen für die alkalische Wäsche als Natronlauge übertragbar.

### Beispiel 1: Dreistufige Wäsche von EHN (erfindungsgemäß)

### Beispiel 1.1: Einstufige saure Wäsche

Ungewaschenes Ethylhexylnitrat (EHN) (134,7 g/min) aus einer kontinuierlichen Nitrierung von Ethylhexanol gemäß EP 1 792 891 A1 mit einem Restgehalt an Schwefelsäure (0,13%), Salpetersäure (0,84 %) und Ethylhexanol (ca. 0,4 %) wurde zusammen mit 67 g/min demineralisiertem Wasser und im Kreis geführter Waschsäure im Verhältnis 1 : 1 mittels eines Strahlmischers (Injektors) mit dem Waschmedium als Zentralstrahl bei Raumtemperatur (ca. 21 °C) in einen Rohrreaktor eingespeist, welcher noch zusätzlich drei statische Mischelemente enthielt. Die Relativgeschwindigkeit zwischen Zentralstrahl und zu waschendem EHN betrug ca. 8 : 1. Die Verweilzeit im Rohrreaktor betrug nicht mehr als 5 Sekunden. Der Druckabfall über die gesamte Rohrreaktorlänge betrug ca. 0,9 bar (entsprechend einer Mischenergie von etwa 90 Joule/I). Nach Phasentrennung enthielt das EHN noch ca. 2.700 ppm an Säure (als Salpetersäure angegeben); das Waschwasser enthielt ca. 0,25 % Schwefelsäure und ca. 1,65 % Salpetersäure.

### Beispiel 1.2: Einstufige alkalische Wäsche

Das aus der sauren Wäsche gemäß Beispiel 1.1 stammende Ethylhexylnitrat (EHN) (133,3 g/min) mit einem Restgehalt an Säure von ca. 2.700 ppm (als Salpetersäure angegeben) wurde zusammen mit 67 g/min einer Ammoniak (0,16 %, d. h. 10 % Überschuss) enthaltenden wässrigen Lösung und im Kreis geführter Waschlauge im Verhältnis 1 : 1 mittels eines Strahlmischers (Injektors) mit dem Waschmedium als Zentralstrahl bei Raumtemperatur (ca. 21 °C) in einen Rohrreaktor eingespeist, welcher noch zusätzlich drei statische Mischelemente enthielt. Die Relativgeschwindigkeit zwischen Zentralstrahl und zu waschendem EHN betrug ca. 8 : 1. Die Verweilzeit im Rohrreaktor betrug nicht mehr als 5 Sekunden. Der Druckabfall über die gesamte Rohrreaktorlänge betrug ca. 0,9 bar (entsprechend einer Mischenergie von etwa 90 Joule/I). Nach Phasentrennung enthielt das EHN noch ca. 29 ppm an Säure (als Salpetersäure angegeben).

### Beispiel 1.3: Einstufige Neutralwäsche

Das aus der alkalischen Wäsche gemäß Beispiel 1.2 stammende Ethylhexylnitrat (EHN) (133 g/min) mit einem Restgehalt an Säure von ca. 29 ppm (als Salpetersäure angegeben) wurde zusammen mit 67 g/demineralisiertem Wasser und im Kreis geführtem Waschwasser im Verhältnis 1 : 1 mittels eines Strahlmischers (Injektors) mit dem Waschmedium als Zentralstrahl bei Raumtemperatur (ca. 21 °C) in einen Rohrreaktor eingespeist, welcher noch zusätzlich drei statische Mischelemente enthielt. Die Relativgeschwindigkeit zwischen Zentralstrahl und zu waschendem EHN betrug ca. 8 : 1. Die Verweilzeit im Rohrreaktor betrug nicht mehr als 5 Sekunden. Der Druckabfall über die gesamte Rohrreaktorlänge betrug ca. 0,9 bar (entsprechend einer Mischenergie von etwa 90 Joule/I). Nach Phasentrennung enthielt das EHN noch ca. 0,1 ppm an Säure (als Salpetersäure angegeben).

### Beispiel 2: Wäsche von EHN (Vergleich)

### Beispiel 2.1: Einstufige alkalische Wäsche

Ethylhexylnitrat (EHN) (133,3 g/min) aus einer sauren Wäsche, wie in Beispiel 1.1 beschrieben, mit einem Restgehalt an Säure von ca. 2.700 ppm (als Salpetersäure angegeben) wurde zusammen mit 67 g/min einer Ammoniak (0,16 %, d. h. 10 % Überschuss) enthaltenden wässrigen Lösung und im Kreis geführter Waschlauge im Verhältnis 1 : 1 mittels eines Strahlmischers (Injektors) mit dem Waschmedium als Zentralstrahl bei Raumtemperatur (ca. 21 °C) in einen Rohrreaktor eingespeist, welcher jedoch keine statischen Mischelemente enthielt. Die Relativgeschwindigkeit zwischen Zentralstrahl und zu waschendem EHN betrug ca. 8 : 1. Die Verweilzeit im Rohrreaktor betrug nicht mehr als 5 Sekunden. Der Druckabfall über die gesamte Rohrreaktorlänge betrug ca. 0,10 bar (entsprechend einer Mischenergie von etwa 10 Joule/I). Nach Phasentrennung enthielt das EHN noch ca. 1.200 ppm an Säure (als Salpetersäure angegeben).

### Beispiel 2.2: Einstufige Neutralwäsche

Das aus der alkalischen Wäsche gemäß Beispiel 2.1 stammende Ethylhexylnitrat (EHN) (133,3 g/min) mit einem Restgehalt an Säure von ca. 1.200 ppm (als Salpetersäure angegeben) wurde zusammen mit 67 g/min demineralisiertem Wasser und im Kreis geführtem Waschwasser im Verhältnis 1 : 1 mittels eines Strahlmischers (Injektors) mit dem Waschmedium als Zentralstrahl bei Raumtemperatur (ca. 21 °C) in einen Rohrreaktor eingespeist, welcher keine statischen Mischelemente enthielt. Die Relativgeschwindigkeit zwischen Zentralstrahl und zu waschendem EHN betrug ca. 8 : 1. Die Verweilzeit im Rohrreaktor betrug nicht mehr als 5 Sekunden. Der Druckabfall über die gesamte Rohrreaktorlänge betrug ca. 0,10 bar (entsprechend einer Mischenergie von etwa 10 Joule/I). Nach Phasentrennung enthielt das EHN noch ca. 456 ppm an Säure (als Salpetersäure angegeben).

### Beispiel 3: Einstufige Wäsche von EHN (erfindungsgemäß)

Ungewaschenes Ethylhexylnitrat (EHN) (134,7 g/min) aus einer kontinuierlichen Nitrierung von Ethylhexanol gemäß EP 1 792 891 A1 mit einem Restgehalt an Schwefelsäure (0,13%), Salpetersäure (0,84 %) und Ethylhexanol (ca. 0,4 %) wurde zusammen mit 67 g/min einer Ammoniak (0,6 %) enthaltenden wässrigen Lösung und im Kreis geführter Waschlauge im Verhältnis 1 : 1 mittels eines Strahlmischers (Injektors) mit dem Waschmedium als Zentralstrahl bei Raumtemperatur (ca. 21 °C) in einen Rohrreaktor eingespeist, welcher noch zusätzlich sechs statische Mischelemente enthielt. Die Relativgeschwindigkeit zwischen Zentralstrahl und zu waschendem EHN betrug ca. 8 : 1. Die Verweilzeit im Rohrreaktor betrug nicht mehr als 5 Sekunden. Der Druckabfall über die gesamte Rohrreaktorlänge betrug ca. 1,8 bar (entsprechend einer Mischenergie von etwa 180 Joule/I). Nach Phasentrennung enthielt das EHN noch ca. 26 ppm an Säure (als Salpetersäure angegeben).

## Patentansprüche

1. Verfahren zur Entfernung von bei der Herstellung von aliphatischen oder cycloaliphatischen Salpetersäureestern (Nitratestern) anfallenden Verunreinigungen, insbesondere zur Entfernung von Verunreinigungen aus bei der Nitrierung von nitrierbaren ein- oder mehrwertigen aliphatischen oder cycloaliphatischen Alkoholen nach Abtrennung der Nitrierendsäure anfallenden Rohsalpetersäureestern, durch Behandlung mit mindestens einem Waschmedium, wobei das Verfahren die folgenden Verfahrensschritte umfasst:
(a) zunächst werden die Rohsalpetersäureester mit einem Waschmedium in Kontakt gebracht, wobei die Rohsalpetersäureester und das Waschmedium derart ineinander verteilt werden, dass eine Emulsion resultiert; und
(b) nachfolgend wird die resultierende Emulsion in einen Rohrreaktor einspeist, wobei der Rohrreaktor mit Mischelementen, insbesondere zum Eintrag von zusätzlicher Mischenergie, ausgestattet ist, so dass während des Durchtritts der Emulsion durch den Rohrreaktor die in den Rohsalpetersäureestern anfänglich vorhandenen Verunreinigungen zumindest teilweise entfernt werden und/oder so dass während des Durchtritts der Emulsion durch den Rohrreaktor die in den Rohsalpetersäureestern anfänglich vorhandenen Verunreinigungen zumindest teilweise in das Waschmedium überführt und/oder hierdurch neutralisiert werden.

2. Verfahren nach Anspruch 1, **dadurch gekennzeichnet, dass** die aufzureinigenden Salpetersäureester ausgewählt sind aus (i) Salpetersäureestern von aliphatischen oder cycloaliphatischen Monoalkoholen, insbesondere von linearen oder verzweigten aliphatischen oder cycloaliphatischen Monoalkoholen, insbesondere von Hexanolen, Heptanolen, Octanolen, Ethylhexylalkohol und Cyclohexanol; (ii) Salpetersäureestern von zweiwertigen aliphatischen Alkoholen, insbesondere von Ethylenglykol und Propylenglykol und deren Oligomeren und Polymeren, wie Ethylen- oder Propylendiglykol, Ethylen- oder Propylentriglykol und Polyethylen- oder Polypropylenglykol; (iii) Salpetersäureestern von dreiwertigen aliphatischen Alkoholen, insbesondere von Trimethylolethan und Glycerin; (iv) Salpetersäureestern von vier- oder mehrwertigen aliphatischen Alkoholen, insbesondere von Pentaerythrit und Dipentaerythrit.

3. Verfahren nach Anspruch 1 oder 2, **dadurch gekennzeichnet,**
**dass** die Mischelemente statische Mischelemente sind und/oder als statische Mischelemente ausgebildet sind; und/oder
**dass** die Mischelemente, insbesondere die statischen Mischelemente, innenseitig im Rohrreaktor fixiert sind, insbesondere mit den Innenwandungen des Rohrreaktors (dauerhaft oder lösbar) verbunden sind; und/oder
**dass** die Mischelemente als Einschubelemente ausgebildet sind, insbesondere wobei die Einschubelemente bedarfsweise nach Anzahl und/oder Position in den Rohrreaktor eingefügt und/oder in dem Rohrreaktor positioniert werden können; und/oder
**dass** die Mischelemente als Bleche, insbesondere Prall- oder Umlenkbleche, als Blenden, als statische Mischer oder als Stromteiler ausgebildet sind; und/oder
**dass** 1 bis 15 Mischelemente, insbesondere 2 bis 15 Mischelemente, bevorzugt 2 bis 10 Mischelemente, besonders bevorzugt 2 bis 5 Mischelemente, im Rohrreaktor angeordnet sind; und/oder
**dass**, insbesondere durch die Mischelemente, im Rohrreaktor eine (volumenbezogene) Mischenergie von 20 bis 1000 Joule/Liter, bevorzugt 25 bis 500 Joule/Liter, besonders bevorzugt 30 bis 200 Joule/Liter, eingetragen wird; und/oder
**dass** der Druckabfall pro Mischelement 0,2 bar bis 3,0 bar, bevorzugt 0,3 bis 1,5 bar, besonders bevorzugt 0,3 bis 0,8 bar, beträgt.

4. Verfahren nach einem der vorangehenden Ansprüche, **dadurch gekennzeichnet, dass** die Herstellung der Emulsion in Schritt (a) mittels einer Dispergiereinrichtung, insbesondere mittels eines Mischorgans, erfolgt;
insbesondere wobei als Dispergiereinrichtung, insbesondere als Mischorgan, ein Strahlmischer (Jet-Mixer) oder eine Pumpe, insbesondere eine Strahlpumpe (Injektor), eingesetzt wird; und/oder
insbesondere wobei die Dispergiereinrichtung, insbesondere das Mischorgan, einen Treibstrahl (Zentralstrahl) und einen den Treibstrahl umgebenden Ringstrahl erzeugt, insbesondere wobei der Treibstrahl durch das Waschmedium und der Ringstrahl durch die aufzureinigenden Rohsalpetersäureester gebildet wird; und/oder insbesondere wobei als Dispergiereinrichtung, insbesondere als Mischorgan, ein Strahlmischer (Jet-Mixer) oder eine Strahlpumpe (Injektor) eingesetzt wird, insbesondere wobei der Strahlmischer oder die Strahlpumpe einen vorzugsweise zentralen Treibstrahl und einen den Treibstrahl umgebendes Medium, insbesondere in Form eines Ringstrahls, erzeugt, insbesondere wobei der Treibstrahl durch das Waschmedium und der Ringstrahl durch die aufzureinigenden Rohsalpetersäureester gebildet wird; und/oder
insbesondere wobei die Dispergiereinrichtung, insbesondere das Mischorgan, einen Treibstrahl (Zentralstrahl) und einen den Treibstrahl umgebenden Ringstrahl erzeugt; insbesondere wobei der Treibstrahl durch das Waschmedium und der Ringstrahl durch die aufzureinigenden Rohsalpetersäureester gebildet wird und/oder insbesondere wobei das Verhältnis der Geschwindigkeiten zwischen dem Treibstrahl einerseits und dem den Treibstrahl umgebenden Ringstrahl andererseits im Bereich von 1 : 6 bis 35 : 1, bevorzugt im Bereich von 1 : 2 bis 25 : 1, besonders bevorzugt im Bereich von 1 : 1 bis 12 : 1, eingestellt wird; und/oder
insbesondere wobei die Dispergiereinrichtung, insbesondere das Mischorgan, dem Rohrreaktor vorgeschaltet, insbesondere unmittelbar vorgeschaltet, ist, insbesondere wobei die Dispergiereinrichtung, insbesondere das Mischorgan, in den Rohrreaktor übergeht; und/oder
insbesondere wobei die Dispergiereinrichtung, insbesondere das Mischorgan, in den Rohrreaktor integriert ist und/oder Bestandteil des Rohrreaktors ist.

5. Verfahren nach einem der vorangehenden Ansprüche, **dadurch gekennzeichnet,**
**dass** die Verweilzeit der aufzureinigenden Rohsalpetersäureester im Rohrreaktor 0,05 bis 130 Sekunden, bevorzugt 0,1 bis 70 Sekunden, besonders bevorzugt 1 bis 35 Sekunden, beträgt; und/oder
**dass** das Masseverhältnis zwischen aufzureinigenden Rohsalpetersäureestern einerseits und Waschmedium, insbesondere frisch zugesetztem Waschmedium, andererseits im Bereich von 250 : 1 bis 1 : 20, bevorzugt im Bereich von 120 : 1 bis 1 : 8, besonders bevorzugt im Bereich von 12 : 1 bis 1 : 3, eingestellt wird; und/oder
**dass** das Masseverhältnis zwischen aufzureinigenden Rohsalpetersäureestern und Waschmedium im Rohrreaktor und/oder Waschapparat im Bereich von 30 : 1 bis 1 : 6, bevorzugt im Bereich von 15 : 1 bis 1 : 5, besonders bevorzugt im Bereich von 6 : 1 bis 1 : 3, eingestellt wird; und/oder
**dass** das Waschmedium unter Verfahrensbedingungen, insbesondere bei Temperaturen ab 5° C, vorzugsweise bei Temperaturen ab 25 °C, und Atmosphärendruck flüssig ist und bevorzugt wässrig basiert ist, vorzugsweise Wasser, ist; und/oder dass das Verfahren kontinuierlich durchgeführt wird; und/oder
**dass** das Verfahren zur Durchführung einer sauren Wäsche und/oder einer basischen Wäsche und/oder einer neutralen Wäsche der aufzureinigenden Rohsalpertersäureester eingesetzt wird.

6. Verfahren nach einem der vorangehenden Ansprüche, **dadurch gekennzeichnet, dass** dem Waschmedium, insbesondere zur Durchführung einer basischen Wäsche, mindestens eine Base zugesetzt wird, insbesondere wobei die Base ausgewählt ist aus der Gruppe von anorganischen Hydroxiden, Carbonaten, Hydrogencarbonaten und Ammoniak sowie deren Mischungen oder Kombinationen, bevorzugt aus der Gruppe von Natronlauge, Kalilauge, Natriumcarbonat, Kaliumcarbonat, Natriumhydrogencarbonat, Kaliumhydrogencarbonat, Ammoniak, Ammoniumcarbonat, sowie deren Mischungen oder Kombinationen, und/oder insbesondere wobei der Gehalt an Base im Waschmedium 0,01 bis 0,4 mol/l, bevorzugt 0,02 bis 0,2 mol/l, beträgt und/ oder insbesondere wobei der Gehalt an Base im Waschmedium mindestens das Doppelte der für die Neutralisation benötigten Alkalimenge aller als Verunreinigungen enthaltener mit Basen Salze bildenden Stoffe beträgt.

7. Verfahren nach einem der vorangehenden Ansprüche, **dadurch gekennzeichnet, dass** das Verfahren, insbesondere die Verfahrensschritte (a) und (b), wiederholt durchgeführt wird und/oder mehrfach ausgeführt wird, wobei die nach jeweils einem Verfahrensdurchlauf resultierenden (teil)aufgereinigten Rohsalpetersäureester erneut in den nachfolgenden Verfahrensdurchlauf eingespeist werden;
insbesondere wobei mindestens ein Verfahrensdurchlauf als basische Wäsche und/oder mit einem basischen Waschmedium durchgeführt wird und mindestens ein hierzu nachgeschalteter Verfahrensdurchlauf als neutrale Wäsche und/oder mit einem neutralen Waschmedium durchgeführt wird; und/oder
insbesondere wobei zwei, vorzugsweise drei nacheinandergeschaltete Verfahrensdurchläufe durchgeführt werden, welche gegebenenfalls eine erste Wäsche mit einem sauren Waschmedium ("saure Wäsche"), hierzu nachgeschaltet eine zweite Wäsche mit einem basischen Waschmedium ("basische Wäsche") und wiederum hierzu nachgeschaltet eine dritte Wäsche mit einem neutralen Waschmedium ("neutrale Wäsche") umfassen.

8. Verfahren nach einem der vorangehenden Ansprüche, **dadurch gekennzeichnet,**
**dass** die aufzureinigenden Rohsalpetersäureester unter Verfahrensbedingungen flüssig sind und/oder dass die aufzureinigenden Rohsalpetersäureester gegebenenfalls in einem inerten Lösungsmittel gelöst vorliegen; und/oder
**dass** sich Verfahrensschritt (b) eine Abtrennung der von den Verunreinigungen befreiten Salpetersäureestern vom Waschmedium, vorzugsweise in einer Phasentrenneinrichtung (Scheider), anschließt; und/oder
**dass** das Waschmedium, insbesondere nach Abtrennung der von den Verunreinigungen befreiten Salpetersäureester vom Waschmedium, rezykliert wird.

9. Verwendung einer Vorrichtung (Anlage) zur Entfernung von bei der Herstellung von aliphatischen oder cycloaliphatischen Salpetersäureestern (Nitratestern) anfallenden Verunreinigungen, insbesondere zur Entfernung von Verunreinigungen aus bei der Nitrierung von nitrierbaren ein- oder mehrwertigen aliphatischen oder cycloaliphatischen Alkoholen nach Abtrennung der Nitrierendsäure anfallenden Rohsalpetersäureestern, durch Behandlung mit mindestens einem Waschmedium, insbesondere zur Durchführung eines Verfahrens nach einem der vorangehenden Ansprüche,
wobei die Vorrichtung die folgenden Einrichtungen aufweist:
(a) mindestens eine Dispergiereinrichtung, insbesondere mindestens ein Mischorgan, zum Inkontaktbringen und Emulgieren von aufzureinigenden Rohsalpetersäureestern einerseits und Waschmedium andererseits;
(b) stromabwärts zur Dispergiereinrichtung angeordnet, einen Rohrreaktor zur Einspeisung der in der Dispergiereinrichtung erzeugten Emulsion von aufzureinigenden Rohsalpetersäureestern einerseits und Waschmedium andererseits, wobei der Rohrreaktor mit Mischelementen zum Eintrag von zusätzlicher Mischenergie ausgestattet ist, so dass während des Durchtritts der Emulsion durch den Rohrreaktor die in den Rohsalpetersäureestern anfänglich vorhandenen Verunreinigungen zumindest teilweise entfernt werden und/oder so dass während des Durchtritts der Emulsion durch den Rohrreaktor die in den Rohsalpetersäureestern anfänglich vorhandenen Verunreinigungen zumindest teilweise in das Waschmedium überführt und/oder hierdurch neutralisiert werden;
(c) stromabwärts zum Rohrreaktor angeordnet, eine Abtrenneinrichtung, insbesondere eine Scheideeinrichtung (Scheider), zur Abtrennung der von den Verunreinigungen befreiten nitrierten Produkte vom Waschmedium angeordnet ist.

10. Verwendung nach Anspruch 9, **dadurch gekennzeichnet,**
**dass** die Mischelemente statische Mischelemente sind und/oder als statische Mischelemente ausgebildet sind; und/oder
**dass** die Mischelemente, insbesondere die statischen Mischelemente, innenseitig im Rohrreaktor fixiert sind, insbesondere mit den Innenwandungen des Rohrreaktors verbunden sind; und/oder
**dass** die Mischelemente als Einschubelemente ausgebildet sind, insbesondere wobei die Einschubelemente bedarfsweise nach Anzahl und/oder Position in den Rohrreaktor eingefügt und/oder in dem Rohrreaktor positioniert werden können; und/oder
**dass** die Mischelemente als Bleche, insbesondere Prall- oder Umlenkbleche, als Blenden, als statische Mischer oder als Stromteiler ausgebildet sind; und/oder
**dass** 1 bis 15 Mischelemente, insbesondere 2 bis 15 Mischelemente, bevorzugt 2 bis 10 Mischelemente, besonders bevorzugt 2 bis 5 Mischelemente, im Rohrreaktor angeordnet sind; und/oder
**dass** insbesondere durch die Mischelemente, im Rohrreaktor eine (volumenbezogene) Mischenergie von 20 bis 1000 Joule/Liter, bevorzugt 25 bis 500 Joule/Liter, besonders bevorzugt 30 bis 200 Joule/Liter, eingetragen wird; und/oder
**dass** der Druckabfall pro Mischelement 0,2 bar bis 3,0 bar, bevorzugt 0,3 bis 1,5 bar, besonders bevorzugt 0,3 bis 0,8 bar, beträgt.

11. Verwendung nach Anspruch 9 oder 10, **dadurch gekennzeichnet,**
**dass** die Dispergiereinrichtung, insbesondere das Mischorgan, als Strahlmischer (Jet-Mixer) oder Pumpe, insbesondere Strahlpumpe (Injektor), ausgebildet ist; und/oder
**dass** die Dispergiereinrichtung, insbesondere das Mischorgan, derart ausgebildet ist, dass sie einen Treibstrahl (Zentralstrahl) und einen den Treibstrahl umgebenden Ringstrahl erzeugt; und/oder
**dass** die Dispergiereinrichtung, insbesondere das Mischorgan, als Strahlmischer (Jet-Mixer) oder Strahlpumpe (Injektor) ausgebildet ist, insbesondere wobei der Strahlmischer oder die Strahlpumpe derart ausgebildet ist, dass ein vorzugsweise zentraler Treibstahl und ein den Treibstrahl umgebendes Medium, insbesondere in Form eines Ringstrahls, erzeugbar ist.

12. Verwendung nach einem der Ansprüche 9 bis 11, **dadurch gekennzeichnet,**
**dass** die Dispergiereinrichtung, insbesondere das Mischorgan, dem Reaktor vorgeschaltet, insbesondere unmittelbar vorgeschaltet, ist, insbesondere wobei die Dispergiereinrichtung, insbesondere das Mischorgan, in den Rohrreaktor übergeht; oder aber
**dass** die Dispergiereinrichtung, insbesondere das Mischorgan, in den Rohrreaktor integriert ist und/oder Bestandteil des Rohrreaktors ist.

13. Verwendung einer Produktionsanlage zur Herstellung von Salpetersäureestern ein- oder mehrwertiger aliphatischer oder cycloaliphatischer Alkohole und/oder zur Nitrierung ein- oder mehrwertiger aliphatischer oder cycloaliphatischer Alkohole mit nachfolgender Aufreinigung der bei der Nitrierung entstehenden nitrierten Rohprodukte,
wobei die Produktionsanlage die folgenden Einheiten umfasst:
(a) eine Nitriereinheit zur Herstellung von Salpetersäureestern ein- oder mehrwertiger aliphatischer oder cycloaliphatischer Alkohole und/oder zur Nitrierung ein- oder mehrwertiger aliphatischer oder cycloaliphatischer Alkohole, insbesondere mit einem oder mehreren entsprechenden Reaktionsbehältern zur Durchführung der Nitrierreaktion(en);
(b) gegebenenfalls, in Produktionslinie stromabwärts zur Nitriereinheit angeordnet, eine Abtrenneinrichtung, insbesondere eine Scheideeinrichtung (Scheider), insbesondere zur Abtrennung der Nitrierendsäure von den nitrierten Rohprodukten (Rohsalpetersäureestern);
(c) in Produktionslinie stromabwärts zur Nitriereinheit und zur gegebenenfalls vorhandenen Abtrenneinrichtung angeordnet, eine Wascheinrichtung zur Durchführung einer Wäsche der nitrierten Rohprodukte, wobei die Wascheinrichtung umfasst:
- mindestens eine Dispergiereinrichtung, insbesondere mindestens ein Mischorgan, zum Inkontaktbringen und Emulgieren von aufzureinigenden Rohsalpetersäureestern einerseits und Waschmedium andererseits; und,
- stromabwärts zur Dispergiereinrichtung angeordnet, einen Rohrreaktor zur Einspeisung der in der Dispergiereinrichtung erzeugten Emulsion von aufzureinigenden Rohsalpetersäureestern einerseits und Waschmedium andererseits, wobei der Rohrreaktor mit Mischelementen zum Eintrag von zusätzlicher Mischenergie ausgestattet ist, so dass während des Durchtritts der Emulsion durch den Rohrreaktor die in den Rohsalpetersäureestern anfänglich vorhandenen Verunreinigungen zumindest teilweise entfernt werden und/oder so dass während des Durchtritts der Emulsion durch den Rohrreaktor die in den Rohsalpetersäureestern anfänglich vorhandenen Verunreinigungen zumindest teilweise in das Waschmedium überführt und/oder hierdurch neutralisiert werden;
(d) gegebenenfalls, in Produktionslinie stromabwärts zur Wascheinrichtung angeordnet, einen Rührkessel, insbesondere zur Erhöhung der Kontakt- und/oder Verweilzeit zwischen Salpetersäureestern einerseits und Waschmedium andererseits;
(e) in Produktionslinie stromabwärts zur Wascheinheit und zum gegebenenfalls vorhandenen Rührkessel angeordnet, eine Abtrenneinrichtung, insbesondere eine Scheideeinrichtung (Scheider), insbesondere zur Abtrennung der von den Verunreinigungen befreiten Salpetersäureester vom Waschmedium.

14. Verwendung nach Anspruch 13, **dadurch gekennzeichnet,**
**dass** die Mischelemente statische Mischelemente sind und/oder als statische Mischelemente ausgebildet sind; und/oder
**dass** die Mischelemente, insbesondere die statischen Mischelemente, innenseitig im Rohrreaktor fixiert sind, insbesondere mit den Innenwandungen des Rohrreaktors verbunden sind; und/oder
**dass** die Mischelemente als Einschubelemente ausgebildet sind, insbesondere wobei die Einschubelemente bedarfsweise nach Anzahl und/oder Position in den Rohrreaktor eingefügt und/oder in dem Rohrreaktor positioniert werden können; und/oder
**dass** die Mischelemente als Bleche, insbesondere Prall- oder Umlenkbleche, als Blenden, als statische Mischer oder als Stromteiler ausgebildet sind; und/oder
**dass** 1 bis 15 Mischelemente, insbesondere 2 bis 15 Mischelemente, bevorzugt 2 bis 10 Mischelemente, besonders bevorzugt 2 bis 5 Mischelemente, im Rohrreaktor angeordnet sind; und/oder
**dass**, insbesondere durch die Mischelemente, im Rohrreaktor eine (volumenbezogene) Mischenergie von 20 bis 1000 Joule/Liter, bevorzugt 25 bis 500 Joule/Liter, besonders bevorzugt 30 bis 200 Joule/Liter, eingetragen wird; und/oder
**dass** der Druckabfall pro Mischelement 0,2 bar bis 3,0 bar, bevorzugt 0,3 bis 1,5 bar, besonders bevorzugt 0,3 bis 0,8 bar, beträgt.

15. Verwendung nach Anspruch 13 oder 14, **dadurch gekennzeichnet,**
**dass** die Dispergiereinrichtung, insbesondere das Mischorgan, als Strahlmischer (Jet-Mixer) oder Pumpe, insbesondere Strahlpumpe (Injektor), ausgebildet ist; und/oder
**dass** die Dispergiereinrichtung, insbesondere das Mischorgan, derart ausgebildet ist, dass sie einen Treibstrahl (Zentralstrahl) und einen den Treibstrahl umgebenden Ringstrahl erzeugt; und/oder
**dass** die Dispergiereinrichtung, insbesondere das Mischorgan, als Strahlmischer (Jet-Mixer) oder Strahlpumpe (Injektor) ausgebildet ist, insbesondere wobei der Strahlmischer oder die Strahlpumpe derart ausgebildet ist, dass ein vorzugsweise zentraler Treibstahl und ein den Treibstrahl umgebendes Medium, insbesondere in Form eines Ringstrahls, erzeugbar ist; und/oder
**dass** die Dispergiereinrichtung, insbesondere das Mischorgan, dem Reaktor vorgeschaltet, insbesondere unmittelbar vorgeschaltet, ist, insbesondere wobei die Dispergiereinrichtung, insbesondere das Mischorgan, in den Rohrreaktor übergeht; und/oder
**dass** die Dispergiereinrichtung, insbesondere das Mischorgan, in den Rohrreaktor integriert ist und/oder Bestandteil des Rohrreaktors ist.

## Claims

1. Method for the removal of impurities arising during the preparation of aliphatic or cycloaliphatic nitric acid esters (nitrate esters), in particular for the removal of impurities from raw nitric acid esters obtained during the nitration of nitratable mono- or polyhydric aliphatic or cycloaliphatic alcohols after separation of the raw nitric acid ester, resulting from the nitrating acid by treatment with at least one washing agent (washing medium), wherein the method comprises the following method steps:
(a) the raw nitric acid esters are first brought into contact with a washing agent, wherein the raw nitric acid esters and the washing agent are so distributed in one another such that an emulsion results; and
(b) subsequently, the resulting emulsion is fed into a tubular reactor, wherein the tubular reactor is provided with mixing elements, in particular for introducing additional mixing energy, such that the impurities initially present in the raw nitric acid esters are at least partially removed during the passage of the emulsion through the tubular reactor and/or such that during the passage of the emulsion through the tubular reactor, the impurities initially present in the raw nitric acid esters are at least partially transferred into the washing agent and/or neutralized thereby.

2. Method according to claim 1,
**characterized in that**
the nitric acid esters to be purified are selected from (i) nitric acid esters of aliphatic or cycloaliphatic monoalcohols, in particular linear or branched aliphatic or cycloaliphatic monoalcohols, in particular of hexanoates, heptanoans, octanols, ethylhexyl alcohol and cyclohexanol; (ii) nitric acid esters of dihydric aliphatic alcohols, in particular ethylene glycol and propylene glycol and their oligomers and polymers, such as ethylene diglycol or propylene diglycol, ethylene triglycol or propylene triglycol, and polyethylene glycol or polypropylene glycol; (iii) nitric acid esters of trivalent aliphatic alcohols, in particular of trimethylolethane and glycerol; (iv) nitric acid esters of tetrahydric or polyhydric aliphatic alcohols, in particular pentaerythritol and dipentaerythritol.

3. Method according to claim 1 or 2,
**characterized in that**
the mixing elements are static mixing elements and/or are formed as static mixing elements; and/or
the mixing elements, in particular the static mixing elements, are fixed on the inside in the tubular reactor, in particular to the inner walls of the tubular reactor (permanently or detachably); and/or
the mixing elements are designed as insertion elements, in particular wherein the insertion elements may be inserted into the tubular reactor and/or positioned in the tubular reactor according to the number and/or position; and/or
the mixing elements are designed as plates, in particular baffle or deflection plates, as diaphragms, as static mixers or as flow dividers; and/or
1 to 15 mixing elements, in particular 2 to 15 mixing elements, preferably 2 to 10 mixing elements, particularly preferably 2 to 5 mixing elements, are arranged in the tubular reactor; and/or
a (volume-related) mixing energy of 20 to 1000 joules/liter, preferably 25 to 500 joules/liter, particularly preferably 30 to 200 joules/liter, is introduced into the tubular reactor, in particular by means of the mixing elements; and/or
the pressure drop per mixing element is 0.2 bar to 3.0 bar, preferably 0.3 to 1.5 bar, particularly preferably 0.3 to 0.8 bar.

4. Method according to one of the preceding claims,
**characterized in that**
the emulsion is prepared in step (a) by means of a dispersing device, in particular by means of a mixing element;
in particular wherein a jet mixer or a pump, in particular a jet pump, is used as a dispersing device, in particular as a mixing member; and/or
in particular wherein the dispersing device, in particular the mixing element, produces a propulsion jet (central jet) and an annular jet surrounding the propulsion jet, in particular wherein the propulsion jet is formed by the washing agent and the annular jet by the raw nitric acid esters to be purified, and/or
in particular wherein a jet mixer or a jet pump (injector) is used as the dispersing device, in particular as the mixing element, in particular wherein the jet mixer or the jet pump produces a preferably central propulsion jet and an agent surrounding the propulsion jet, in particular in the form of an annular jet, in particular wherein the propulsion jet is formed by the washing agent and the annular jet by the raw nitric acid esters to be purified; and/or
in particular wherein the dispersing device, in particular the mixing element, produces propulsion jet (central jet) and an annular jet surrounding the propulsion jet, wherein the propulsion jet is formed by the washing agent and the annular jet by the raw nitric acid esters to be purified and/or in particular wherein the ratio of the velocities between the propulsion jet, on the one hand, and the annular jet surrounding the propulsion jet, on the other hand, is adjusted in the range from 1: 6 to 35 : 1, preferably in the range from 1 : 2 to 25 : 1, particularly preferably in the range from 1 : 1 to 12 : 1, and/or
in particular wherein the dispersing device, in particular the mixing element, is connected upstream of the tubular reactor, in particular directly upstream, in particular wherein the dispersing device, in particular the mixing element, passes into the tube reactor; and/or
in particular wherein the dispersing device, in particular the mixing element, is integrated into the tubular reactor and/or is a component of the tubular reactor.

5. Method according to one of the preceding claims,
**characterized in that**
the residence time of the raw nitric acid esters to be purified in the tubular reactor is 0.05 to 130 seconds, preferably 0.1 to 70 seconds, more preferably 1 to 35 seconds; and/or
the mass ratio between the nitric acid esters to be purified, on the one hand, and the washing agent, in particular freshly added washing agent, on the other hand, lies in the range from 250 : 1 to 1 : 20, preferably in the range from 120 : 1 to 1 : 8, more preferably in the range from 12 : 1 to 1 : 3; and/or
the mass ratio between the nitric acid esters to be purified and the washing agent in the tubular reactor and/or washing apparatus is adjusted in the range from 30 : 1 to 1 : 6, preferably in the range from 15 : 1 to 1 : 5, particularly preferably in the range from 6 : 1 to 1 : 3; and/or
the washing agent is liquid under method conditions, in particular at temperatures above 5 °C, preferably at temperatures above 25 °C and at atmospheric pressure, and is preferably water-based, preferably water; and/or
the method is carried out continuously; and/or
the method is used for carrying out an acidic wash and/or a basic wash and/or a neutral wash of the raw acid esters to be purified.

6. Method according to one of the preceding claims,
**characterized in that**
a base is added to the washing agent, in particular for performing a basic wash, in particular wherein the base is selected from the group consisting of inorganic hydroxides, carbonates, hydrogencarbonates and ammonia, as well as mixtures or combinations thereof, preferably from the group consisting of sodium hydroxide solution, potassium hydroxide solution, sodium carbonate, potassium carbonate, sodium hydrogencarbonate, potassium hydrogencarbonate, ammonia, ammonium carbonate as well as mixtures or combinations thereof, and/or in particular wherein the content of base in the washing agent is from 0.01 to 0.4 mol/l, preferably from 0.02 to 0.2 mol/l, and/or in particular wherein the content of base in the washing agent is at least double the amount of alkali metal salts required for the neutralization of all substances formed as impurities and forming base salts.

7. Method according to one of the preceding claims,
**characterized in that**
the method, in particular method steps (a) and (b), is/are repeated and/or carried out several times, wherein the (partially) purified nitric acid esters resulting from a respective method cycle are fed back into the subsequent method cycle;
in particular wherein at least one method cycle is carried out as basic washing and/or with a basic washing agent, and at least one subsequent method cycle is carried out as neutral washing and/or with a neutral washing agent; and/or
in particular two, preferably three subsequent method cycles are carried out, which optionally comprise ("acidic wash") followed by a second wash with a basic washing agent ("basic wash") followed again by a third wash with a neutral washing agent ("neutral wash").

8. Method according to one of the preceding claims,
**characterized in that**
the raw nitric acid esters to be purified are subject to conditions and/or the raw nitric acid esters to be purified are optionally dissolved in an inert solvent; and/or
in method step (b), separation of the nitric acid esters freed from the impurities from the washing agent, preferably in a phase separation device (separator); and/or
the washing agent is recycled, in particular after separation of the nitric acid esters, which are freed from the impurities from the washing agent.

9. Use of a device (plant) for the removal of impurities arising during the preparation of aliphatic or cycloaliphatic nitric acid esters (nitrate esters), in particular for the removal of impurities from aliphatic or cycloaliphatic alcohols obtainable during nitration of monovalent or polyvalent aliphatic or cycloaliphatic alcohols after removal of the raw nitric acid esters resulting from the nitrating acid, by treatment with at least one washing agent, in particular for carrying out a method according to one of the preceding claims,
wherein the apparatus comprises the following means:
(a) at least one dispersing device, in particular at least one mixing element, for contacting and emulsifying raw nitric acid esters to be purified, on the one hand, and washing agent on the other hand;
(b) downstream of the dispersing device is arranged a tubular reactor for supplying the emulsion of nitric acid esters to be purified in the dispersing device, on the one hand, and washing agent on the other hand, wherein the tubular reactor is provided with mixing elements for the introduction of additional mixing energy, so that, during the passage of the emulsion through the tubular reactor, the impurities initially present in the nitric acid esters are at least partially removed and/or so that, during the passage of the emulsion through the tubular reactor, the impurities initially present in the nitric acid esters are at least partially converted into the washing agent and/or neutralized thereby;
(c) downstream of the tubular reactor is arranged a separation device, in particular a separation device (separator) to separate the nitrated products, which are freed from the impurities from the washing agent.

10. Use according to claim 9,
**characterized in that**
the mixing elements are static mixing elements and/or are designed as static mixing elements; and/or
the mixing elements, in particular the static mixing elements, are fixed on the inside in the tube reactor, in particular connected to the internal walls of the tubular reactor; and/or
the mixing elements are designed as insertion elements, in particular wherein the insertion elements may be inserted into the tubular reactor and/or positioned in the tubular reactor according to the number and/or position; and/or
the mixing elements are designed as plates, in particular baffle or deflection plates, as diaphragms, as static mixers or as flow dividers; and/or
1 to 15 mixing elements, in particular 2 to 15 mixing elements, preferably 2 to 10 mixing elements, particularly preferably 2 to 5 mixing elements, are arranged in the tubular reactor; and/or
in particular, through the mixing elements, a (volume-related) mixing energy of 20 to 1000 joules/liter, preferably 25 to 500 joules/liter, particularly preferably 30 to 200 joules/liter, is introduced into the tubular reactor; and/or
the pressure drop per mixing element is 0.2 bar to 3.0 bar, preferably 0.3 to 1.5 bar, particularly preferably 0.3 to 0.8 bar.

11. Method according to claim 9 or 10,
**characterized in that**
the dispersing device, in particular the mixing element, is designed as a jet mixer or a pump, in particular a jet pump (injector); and/or
the dispersing device, in particular the mixing element, is designed in such a way that it produces a propulsion jet (central jet) and an annular jet surrounding the propulsion jet; and/or
the dispersing device, in particular the mixing element, is designed as a jet mixer or jet pump (injector), in particular wherein the jet mixer or the jet pump is so designed that a preferably central propulsion jet and a medium surrounds the propulsion jet, in particular in the form of an annular jet.

12. Method according to one of the claims 9 to 11,
**characterized in that**
the dispersing device, in particular the mixing element, is connected upstream of the reactor, in particular directly upstream, in particular wherein the dispersing device, in particular the mixing element, passes into the tubular reactor; or else
the dispersing device, in particular the mixing element, is integrated into the tubular reactor and/or is a component of the tubular reactor.

13. Use of a production plant for the production of nitric acid esters of monohydric or polyhydric aliphatic or cycloaliphatic alcohols and/or for the nitration of monohydric or polyhydric aliphatic or cycloaliphatic alcohols with subsequent purification of the nitrided raw products formed during the nitration,
wherein the production plant comprises the following units:
(a) a nitration unit for the preparation of nitric acid esters of monohydric or polyhydric aliphatic or cycloaliphatic alcohols and/or for the nitration of monohydric or polyhydric aliphatic or cycloaliphatic alcohols, in particular with one or more corresponding reaction vessels for carrying out the nitriding reaction(s);
(b) optionally, in the production line downstream from the nitrating unit, a dividing device, in particular a separation device (separator), in particular for separation of the nitrating acid from the nitrided raw products (raw nitric acid esters);
(c) in the production line downstream of the nitriding unit and the optionally provided separation device, a washing device for carrying out washing of the nitrided raw products, wherein the washing device comprises:
- at least one dispersing device, in particular at least one mixing element, for contacting and emulsifying raw nitric acid esters to be purified, on the one hand, and washing agent, on the other hand; and,
- downstream of the dispersing device is arranged a tubular reactor for the introduction of emulsion produced from the raw nitric acid esters in the dispersing device, on the one hand, and the washing agent, on the other hand, wherein the tubular reactor is provided with mixing elements for the introduction of additional mixing energy, so that, during the passage of the emulsion through the tubular reactor, the impurities initially present in the raw nitric acid esters are at least partially removed, and/or so that during the passage of the emulsion through the tubular reactor, the impurities initially present in the raw nitric acid esters are at least partly transferred into the washing agent and/or neutralized thereby;
(d) if appropriate, a stirrer tank is arranged downstream of the washing device in the production line, in particular for increasing the contact and/or residence time between nitric acid esters, on the one hand, and the washing agent, on the other hand;
(e) a stirrer tank is optionally arranged downstream of the washing device in the production line and, in particular a separation device (separator), in particular for separating the nitric acid esters, which are freed from the impurities from the washing agent.

14. Use according to claim 13,
**characterized in that**
the mixing elements are static mixing elements and/or are designed as static mixing elements; and/or
the mixing elements, in particular the static mixing elements, are fixed on the inside in the tubular reactor, in particular to the inner walls of the tubular reactor; and/or
the mixing elements are designed as insertion elements, in particular wherein the insertion elements may be inserted into the tubular reactor and/or positioned in the tubular reactor according to the number and/or position; and/or
the mixing elements are designed as plates, in particular baffle or deflection plates, as diaphragms, as static mixers or as flow dividers; and/or
1 to 15 mixing elements, in particular 2 to 15 mixing elements, preferably 2 to 10 mixing elements, particularly preferably 2 to 5 mixing elements, are arranged in the tubular reactor; and/or
a volume-related mixing energy of 20 to 1000 joules/liter, preferably 25 to 500 joules/liter, particularly preferably 30 to 200 joules/liter, is introduced into the tubular reactor, in particular through the mixing elements; and/or
the pressure drop per mixing element is 0.2 bar to 3.0 bar, preferably 0.3 to 1.5 bar, particularly preferably 0.3 to 0.8 bar.

15. Use according to claim 13 or 14,
**characterized in that**
the dispersing device, in particular the mixing element, is designed as a jet mixer or a pump, in particular a jet pump (injector); and/or
the dispersing device, in particular the mixing element, is designed so that it produces a propulsion jet (central jet) and an annular jet surrounding the propulsion jet; and/or
the dispersing device, in particular the mixing element, is designed as a jet mixer or jet pump, in particular wherein the jet mixer or the jet pump (injector) are so designed that a preferably central propulsion jet and a medium surrounding the propulsion jet, in particular in the form of an annular jet, may be generated; and/or
the dispersing device, in particular the mixing element, is connected upstream of the reactor, in particular directly upstream, in particular wherein the dispersing device, in particular the mixing element, passes into the tubular reactor; and/or
the dispersing device, in particular the mixing element, is integrated into the tubular reactor and/or is a component of the tubular reactor.

## Revendications

1. Procédé d'élimination des impuretés résultant de la production d'esters aliphatiques ou cycloaliphatiques de l'acide nitrique (esters nitrates), en particulier d'élimination d'impuretés à partir des esters nitrates bruts résultant de la nitration d'alcools aliphatiques ou cycloaliphatiques uni- ou plurivalents nitrables après séparation de l'acide nitrant, par traitement avec au moins un milieu de lavage, ledit procédé comprenant les étapes suivantes :
(a) en premier lieu, on met les esters nitrates bruts en contact avec un milieu de lavage, les esters nitrates bruts et le milieu de lavage étant dispersés les uns dans l'autre de façon à obtenir une émulsion ; et
(b) ensuite, on introduit l'émulsion ainsi obtenue dans un réacteur tubulaire, lequel réacteur tubulaire est équipé d'éléments mélangeurs, en particulier servant à apporter de l'énergie de mélangeage supplémentaire, de sorte que, lors du passage de l'émulsion dans le réacteur tubulaire, les impuretés initialement présentes dans les esters nitrates bruts sont au moins en partie éliminées, et/ou de sorte que, lors du passage de l'émulsion dans le réacteur tubulaire, les impuretés initialement présentes dans les esters nitrates bruts sont au moins en partie transférées dans le milieu de lavage et/ou neutralisées par celui-ci.

2. Procédé selon la revendication 1, **caractérisé en ce que** les esters nitrates à purifier sont choisis parmi (i) des esters nitrates de monoalcools aliphatiques ou cycloaliphatiques, en particulier de monoalcools aliphatiques ou cycloaliphatiques linéaires ou ramifiés, en particulier d'hexanols, heptanols, octanols, alcool éthylhexylique et cyclohexanol ; (ii) des esters nitrates d'alcools aliphatiques divalents, en particulier d'éthylène glycol et de propylène glycol et leurs oligomères et polymères tels que l'éthylène ou propylène diglycol, l'éthylène ou propylène triglycol et le polyéthylène ou polypropylène glycol ; (iii) des esters nitrates d'alcools aliphatiques trivalents, en particulier de triméthyloléthane et de glycérol ; (iv) des esters nitrates d'alcools aliphatiques tétravalents ou plurivalents, en particulier de pentaérythritol et de dipentaérythritol.

3. Procédé selon la revendication 1 ou la revendication 2, **caractérisé**
**en ce que** les éléments mélangeurs sont des éléments mélangeurs statiques et/ou sont réalisés sous la forme d'éléments mélangeurs statiques ; et/ou
**en ce que** les éléments mélangeurs, en particulier les éléments mélangeurs statiques, sont fixés côté intérieur dans le réacteur tubulaire, en particulier reliés (de manière permanente ou démontable) aux parois internes du réacteur tubulaire ; et/ou
**en ce que** les éléments mélangeurs sont réalisés sous la forme d'éléments rapportés, les éléments rapportés pouvant en particulier être insérés dans le réacteur tubulaire et/ou positionnés dans le réacteur tubulaire en fonction des besoins en termes de quantité et/ou de position ; et/ou
**en ce que** les éléments mélangeurs sont réalisés sous la forme de tôles, en particulier de chicanes ou de tôles déflectrices, de diaphragmes, de mélangeurs statiques ou de diviseurs d'écoulement ; et/ou
**en ce que** 1 à 15 éléments mélangeurs, en particulier 2 à 15 éléments mélangeurs, de préférence 2 à 10 éléments mélangeurs, de façon particulièrement préférée 2 à 5 éléments mélangeurs, sont disposés dans le réacteur tubulaire ; et/ou
**en ce qu'**une énergie de mélangeage (par unité de volume) de 20 à 1000 joules/litre, de préférence 25 à 500 joules/litre, de façon particulièrement préférée 30 à 200 joules/litre, est fournie au réacteur tubulaire, en particulier par les éléments mélangeurs ; et/ou
**en ce que** la chute de pression par élément mélangeur va de 0,2 bar à 3,0 bar, de préférence de 0,3 à 1,5 bar, de façon particulièrement préférée de 0,3 à 0,8 bar.

4. Procédé selon l'une des revendications précédentes, **caractérisé en ce que** la préparation de l'émulsion à l'étape (a) se fait au moyen d'un dispositif de dispersion, en particulier au moyen d'un organe mélangeur ;
dans lequel en particulier le dispositif de dispersion, en particulier l'organe mélangeur, est un mélangeur à jets (jet mixer) ou une pompe, en particulier une pompe à jets (injecteur) ; et/ou
dans lequel en particulier le dispositif de dispersion, en particulier l'organe mélangeur, génère un jet propulseur (jet central) et un jet annulaire entourant le jet propulseur, dans lequel en particulier le jet propulseur est formé par le milieu de lavage et le jet annulaire est formé par les esters nitrates bruts à purifier ; et/ou
dans lequel en particulier le dispositif de dispersion, en particulier l'organe mélangeur utilisé est un mélangeur à jets (jet mixer) ou une pompe à jets (injecteur), dans lequel en particulier le mélangeur à jets ou la pompe à jets génère un jet propulseur de préférence central et un milieu qui entoure le jet propulseur, en particulier sous la forme d'un jet annulaire, dans lequel en particulier le jet propulseur est formé par le milieu de lavage et le jet annulaire est formé par les esters nitrates bruts à purifier ; et/ou
dans lequel en particulier le dispositif de dispersion, en particulier l'organe mélangeur, génère un jet propulseur (jet central) et un jet annulaire entourant le jet propulseur, dans lequel en particulier le jet propulseur est formé par le milieu de lavage et le jet annulaire est formé par les esters nitrates bruts à purifier et/ou dans lequel en particulier le ratio des vitesses entre le jet propulseur d'une part et le jet annulaire entourant le jet propulseur d'autre part est ajusté dans la plage de 1:6 à 35:1, de préférence dans la plage de 1:2 à 25:1, de façon particulièrement préférée dans la plage de 1:1 à 12:1 ; et/ou
dans lequel en particulier le dispositif de dispersion, en particulier l'organe mélangeur, est installé en amont du réacteur tubulaire, en particulier directement en amont, dans lequel en particulier le dispositif de dispersion, en particulier l'organe mélangeur, se prolonge dans le réacteur tubulaire ; et/ou
dans lequel en particulier le dispositif de dispersion, en particulier l'organe mélangeur, est intégré dans le réacteur tubulaire et/ou fait partie intégrante du réacteur tubulaire.

5. Procédé selon l'une des revendications précédentes, **caractérisé**
**en ce que** le temps de séjour des esters nitrates bruts à purifier dans le réacteur tubulaire va de 0,05 à 130 secondes, de préférence de 0,1 à 70 secondes, de façon particulièrement préférée de 1 à 35 secondes ; et/ou
**en ce que** le ratio massique entre les esters nitrates bruts à purifier d'une part et le milieu de lavage, en particulier le milieu de lavage fraîchement ajouté, d'autre part est ajusté dans la plage de 250:1 à 1:20, de préférence dans la plage de 120:1 à 1:8, de façon particulièrement préférée dans la plage de 12:1 à 1:3 ; et/ou
**en ce que** le ratio massique entre les esters nitrates bruts à purifier et le milieu de lavage dans le réacteur tubulaire et/ou l'appareil de lavage est ajusté dans la plage de 30:1 à 1:6, de préférence dans la plage de 15:1 à 1:5, de façon particulièrement préférée dans la plage de 6:1 à 1:3 ; et/ou
**en ce que** le milieu de lavage est liquide dans les conditions du procédé, en particulier à des températures à partir de 5 °C, de préférence à des températures à partir de 25 °C et à la pression atmosphérique, et est de préférence à base aqueuse, de préférence est de l'eau ; et/ou
**en ce que** le procédé s'effectue en continu ; et/ou
**en ce que** le procédé est utilisé pour effectuer un lavage acide et/ou un lavage basique et/ou un lavage neutre des esters nitrates bruts à purifier.

6. Procédé selon l'une des revendications précédentes, **caractérisé en ce que** l'on ajoute au moins une base au milieu de lavage, en particulier pour effectuer un lavage basique, en particulier dans lequel la base est choisie dans le groupe constitué par des hydroxydes inorganiques, des carbonates, des hydrogénocarbonates et l'ammoniaque, ainsi que leurs mélanges ou combinaisons, de préférence dans le groupe constitué par une solution d'hydroxyde de sodium, une solution d'hydroxyde de potassium, le carbonate de sodium, le carbonate de potassium, l'hydrogénocarbonate de sodium, l'hydrogénocarbonate de potassium, l'ammoniaque, le carbonate d'ammonium, ainsi que leurs mélanges ou combinaisons, et/ou dans lequel en particulier la teneur en base dans le milieu de lavage va de 0,01 à 0,4 mol/l, de préférence de 0,02 à 0,2 mol/l, et/ou dans lequel en particulier la teneur en base dans le milieu de lavage est au moins égale à deux fois la quantité d'alcali nécessaire pour neutraliser toutes les substances contenues en tant qu'impuretés et formant des sels avec des bases.

7. Procédé selon l'une des revendications précédentes, **caractérisé en ce que** le procédé, en particulier les étapes de procédé (a) et (b), sont effectuées de manière répétitive et/ou à plusieurs reprises, les esters nitrates bruts (partiellement) purifiés obtenus à chaque fois après un cycle de procédé étant réintroduits dans le cycle de procédé suivant ;
dans lequel en particulier au moins un cycle de procédé est effectué sous la forme d'un lavage basique et/ou avec un milieu de lavage basique et au moins un cycle de procédé consécutif à celui-ci est effectué sous la forme d'un lavage neutre et/ou avec un milieu de lavage neutre ; et/ou
dans lequel en particulier deux, de préférence trois cycles de procédé consécutifs sont effectués, comprenant le cas échéant un premier lavage avec un milieu de lavage acide ("lavage acide"), suivi d'un deuxième lavage avec un milieu de lavage basique ("lavage basique"), lui-même suivi d'un troisième lavage avec un milieu de lavage neutre ("lavage neutre").

8. Procédé selon l'une des revendications précédentes, **caractérisé**
**en ce que** les esters nitrates bruts à purifier sont liquides dans les conditions du procédé et/ou en ce que les esters nitrates bruts à purifier sont présents le cas échéant en solution dans un solvant inerte ;
**en ce que** l'étape de procédé (b) est suivie d'une séparation des esters nitrates débarrassés des impuretés d'avec le milieu de lavage, de préférence dans un dispositif de séparation de phases (séparateur) ; et/ou
**en ce que** le milieu de lavage, en particulier après la séparation des esters nitrates débarrassés des impuretés d'avec le milieu de lavage, est recyclé.

9. Utilisation d'un dispositif (une installation) pour éliminer des impuretés résultant de la production d'esters aliphatiques ou cycloaliphatiques de l'acide nitrique (esters nitrates), en particulier pour éliminer des impuretés d'esters nitrates bruts résultant de la nitration d'alcools aliphatiques ou cycloaliphatiques uni- ou plurivalents nitrables après séparation de l'acide nitrant, par traitement avec au moins un milieu de lavage, en particulier pour mettre en oeuvre un procédé selon l'une des revendications précédentes, ledit dispositif comportant les équipements suivants :
(a) au moins un dispositif de dispersion, en particulier au moins un organe mélangeur, pour mettre en contact et émulsionner les esters nitrates bruts à purifier d'une part et le milieu de lavage d'autre part ;
(b) un réacteur tubulaire, disposé en aval du dispositif de dispersion, pour alimenter l'émulsion d'esters nitrates bruts à purifier d'une part et du milieu de lavage d'autre part produite dans le dispositif de dispersion, lequel réacteur tubulaire est équipé d'éléments mélangeurs pour apporter de l'énergie de mélangeage supplémentaire de sorte que, lors du passage de l'émulsion dans le réacteur tubulaire, les impuretés initialement présentes dans les esters nitrates bruts sont au moins en partie éliminées et/ou de sorte que, lors du passage de l'émulsion dans le réacteur tubulaire, les impuretés initialement présentes dans les esters nitrates bruts sont au moins en partie transférées dans le milieu de lavage et/ou neutralisées par celui-ci ;
(c) un dispositif d'extraction, en particulier un dispositif de séparation (séparateur), disposé en aval du réacteur tubulaire, pour séparer les produits nitrés débarrassés des impuretés d'avec le milieu de lavage.

10. Utilisation selon la revendication 9, **caractérisée**
**en ce que** les éléments mélangeurs sont des éléments mélangeurs statiques et/ou sont réalisés sous la forme d'éléments mélangeurs statiques ; et/ou
**en ce que** les éléments mélangeurs, en particulier les éléments mélangeurs statiques, sont fixés côté intérieur dans le réacteur tubulaire, en particulier sont reliés aux parois internes du réacteur tubulaire ; et/ou
**en ce que** les éléments mélangeurs sont réalisés sous la forme d'éléments rapportés, les éléments rapportés pouvant en particulier être insérés dans le réacteur tubulaire et/ou positionnés dans le réacteur tubulaire en fonction des besoins en termes de quantité et/ou de position ; et/ou
**en ce que** les éléments mélangeurs sont réalisés sous la forme de tôles, en particulier de chicanes ou de tôles déflectrices, de diaphragmes, de mélangeurs statiques ou de diviseurs d'écoulement ; et/ou
**en ce que** 1 à 15 éléments mélangeurs, en particulier 2 à 15 éléments mélangeurs, de préférence 2 à 10 éléments mélangeurs, de façon particulièrement préférée 2 à 5 éléments mélangeurs, sont disposés dans le réacteur tubulaire ; et/ou
**en ce qu'**une énergie de mélangeage (par unité de volume) de 20 à 1000 joules/litre, de préférence 25 à 500 joules/litre, de façon particulièrement préférée 30 à 200 joules/litre, est introduite dans le réacteur tubulaire, en particulier par les éléments mélangeurs ; et/ou
**en ce que** la chute de pression par élément mélangeur va de 0,2 bar à 3,0 bar, de préférence de 0,3 à 1,5 bar, de façon particulièrement préférée de 0,3 à 0,8 bar.

11. Utilisation selon la revendication 9 ou la revendication 10, **caractérisée**
**en ce que** le dispositif de dispersion, en particulier l'organe mélangeur, est réalisé sous la forme d'un mélangeur à jets (jet mixer) ou d'une pompe, en particulier d'une pompe à jets (injecteur) ; et/ou
**en ce que** le dispositif de dispersion, en particulier l'organe mélangeur, est réalisé de telle façon qu'il génère un jet propulseur (jet central) et un jet annulaire entourant le jet propulseur ; et/ou
**en ce que** le dispositif de dispersion, en particulier l'organe mélangeur, est réalisé sous la forme d'un mélangeur à jets (jet mixer) ou d'une pompe à jets (injecteur), le mélangeur à jets ou la pompe à jets étant en particulier réalisé de façon à pouvoir générer un jet propulseur de préférence central et un milieu entourant le jet propulseur, en particulier sous la forme d'un jet annulaire.

12. Utilisation selon l'une des revendications 9 à 11, **caractérisée**
**en ce que** le dispositif de dispersion, en particulier l'organe mélangeur, est disposé en amont du réacteur, en particulier directement en amont, le dispositif de dispersion, en particulier l'organe mélangeur, se prolongeant en particulier dans le réacteur tubulaire ; ou alors
**en ce que** le dispositif de dispersion, en particulier l'organe mélangeur, est intégré dans le réacteur tubulaire et/ou fait partie intégrante du réacteur tubulaire.

13. Utilisation d'une installation de production d'esters nitrates d'alcools aliphatiques ou cycloaliphatiques uni- ou plurivalents et/ou de nitration d'alcools aliphatiques ou cycloaliphatiques uni- ou plurivalents suivie de la purification des produits bruts nitrés résultant de la nitration, l'installation de production comprenant les unités suivantes :
(a) une unité de nitration pour produire des esters nitrates d'alcools aliphatiques ou cycloaliphatiques uni- ou plurivalents et/ou pour nitrer des alcools aliphatiques ou cycloaliphatiques uni- ou plurivalents, en particulier comportant une ou plusieurs cuves de réaction correspondantes pour effectuer la ou les réactions de nitration ;
(b) le cas échéant, disposé dans la chaîne de production en aval de l'unité de nitration, un dispositif d'extraction, en particulier un dispositif de séparation (séparateur), en particulier pour séparer l'acide nitrant des produits bruts nitrés (esters nitrates bruts) ;
(c) disposé dans la chaîne de production en aval de l'unité de nitration et du dispositif d'extraction le cas échéant présent, un dispositif de lavage pour effectuer un lavage des produits bruts nitrés, ledit dispositif de lavage comprenant :
- au moins un dispositif de dispersion, en particulier au moins un organe mélangeur, pour mettre en contact et émulsionner des esters nitrates bruts à purifier d'une part et le milieu de lavage d'autre part ; et,
- disposé en aval du dispositif de dispersion, un réacteur tubulaire pour alimenter l'émulsion, produite dans le dispositif de dispersion, des esters nitrates bruts à purifier d'une part et du milieu de lavage d'autre part, le réacteur tubulaire étant équipé d'éléments mélangeurs pour apporter de l'énergie de mélangeage supplémentaire de sorte que, lors du passage de l'émulsion dans le réacteur tubulaire, les impuretés initialement présentes dans les esters nitrates bruts sont au moins en partie éliminées et/ou de sorte que, lors du passage de l'émulsion dans le réacteur tubulaire, les impuretés initialement présentes dans les esters nitrates bruts sont au moins en partie transférées dans le milieu de lavage et/ou neutralisées par celui-ci ;
(d) le cas échéant, disposée dans la chaîne de production en aval du dispositif de lavage, une cuve d'agitation, en particulier pour augmenter le temps de contact et/ou de séjour entre les esters nitrates d'une part et le milieu de lavage d'autre part ;
(e) disposé dans la chaîne de production en aval de l'unité de lavage et de la cuve d'agitation le cas échéant présente, un dispositif d'extraction, en particulier un dispositif de séparation (séparateur), en particulier pour séparer les esters nitrates débarrassés des impuretés d'avec le milieu de lavage.

14. Utilisation selon la revendication 13, **caractérisée**
**en ce que** les éléments mélangeurs sont des éléments mélangeurs statiques et/ou sont réalisés sous la forme d'éléments mélangeurs statiques ; et/ou
**en ce que** les éléments mélangeurs, en particulier les éléments mélangeurs statiques, sont fixés côté intérieur dans le réacteur tubulaire, en particulier sont reliés aux parois internes du réacteur tubulaire ; et/ou
**en ce que** les éléments mélangeurs sont réalisés sous la forme d'éléments rapportés, ces éléments rapportés pouvant en particulier être insérés dans le réacteur tubulaire et/ou positionnés dans le réacteur tubulaire en fonction des besoins en termes de quantité et/ou de position ; et/ou
**en ce que** les éléments mélangeurs sont réalisés sous la forme de tôles, en particulier de chicanes ou de tôles déflectrices, de diaphragmes, de mélangeurs statiques ou de diviseurs d'écoulement ; et/ou
**en ce que** 1 à 15 éléments mélangeurs, en particulier 2 à 15 éléments mélangeurs, de préférence 2 à 10 éléments mélangeurs, de façon particulièrement préférée 2 à 5 éléments mélangeurs, sont disposés dans le réacteur tubulaire ; et/ou
**en ce qu'**une énergie de mélangeage (par unité de volume) de 20 à 1000 joules/litre, de préférence 25 à 500 joules/litre, de façon particulièrement préférée 30 à 200 joules/litre, est introduite dans le réacteur tubulaire, en particulier par les éléments mélangeurs ; et/ou
**en ce que** la chute de pression par élément mélangeur va de 0,2 bar à 3,0 bar, de préférence de 0,3 à 1,5 bar, de façon particulièrement préférée de 0,3 à 0,8 bar.

15. Utilisation selon la revendication 13 ou la revendication 14, **caractérisée**
**en ce que** le dispositif de dispersion, en particulier l'organe mélangeur, est réalisé sous la forme d'un mélangeur à jets (jet mixer) ou d'une pompe, en particulier d'une pompe à jets (injecteur) ; et/ou
**en ce que** le dispositif de dispersion, en particulier l'organe mélangeur, est réalisé de telle façon qu'il génère un jet propulseur (jet central) et un jet annulaire entourant le jet propulseur ; et/ou
**en ce que** le dispositif de dispersion, en particulier l'organe mélangeur, est réalisé sous la forme d'un mélangeur à jets (jet mixer) ou d'une pompe à jets (injecteur), le mélangeur à jets ou la pompe à jets étant en particulier réalisé de façon à pouvoir générer un jet propulseur de préférence central et un milieu entourant le jet propulseur, en particulier sous la forme d'un jet annulaire ; et/ou
**en ce que** le dispositif de dispersion, en particulier l'organe mélangeur, est disposé en amont du réacteur, en particulier directement en amont, le dispositif de dispersion, en particulier l'organe mélangeur, se prolongeant en particulier dans le réacteur tubulaire ; et/ou
**en ce que** le dispositif de dispersion, en particulier l'organe mélangeur, est intégré dans le réacteur tubulaire et/ou fait partie intégrante du réacteur tubulaire.
